# EUROPEAN PATENT APPLICATION

(11) **EP 4 019 970 A1**
(43) Date of publication of application: **29.06.2022**
(21) Application number: 20217081.7
(22) Date of filing: 23.12.2020
(51) Int. Cl.: G01N 33/543, G01N 33/569, G01N 33/58

(54) **LIPOSOME-RECEPTOR-ASSAY FOR DETECTING NEUTRALIZING ANTIBODIES**

(71) Applicant: Universität Regensburg, 93053 Regensburg (DE)
(72) Inventor: The designation of the inventor has not yet been filed
(74) Representative: Engelhard, Markus

(57) **Abstract**

The present invention relates to a particle collection for detecting a pathogen-neutralizing molecule. The present invention further relates to a composition comprising a particle collection. The present invention also relates to a method of detecting a pathogen-neutralizing molecule. Furthermore, the present invention relates to a kit for detecting a pathogen-neutralizing molecule. The present invention further relates to a point-of-care device, and to a use of a particle collection or a composition in a method of detecting a pathogen-neutralizing molecule.

## Description

### FIELD OF THE INVENTION

The present invention relates to a particle collection for detecting a pathogen-neutralizing molecule. The present invention further relates to a composition comprising a particle collection. The present invention also relates to a method of detecting a pathogen-neutralizing molecule. Furthermore, the present invention relates to a kit for detecting a pathogen-neutralizing molecule. The present invention further relates to a point-of-care device, and to a use of a particle collection or a composition in a method of detecting a pathogen-neutralizing molecule.

### BACKGROUND OF THE INVENTION

It is an immense analytical challenge to assess the functional immune status of patients in a rapid and low-cost fashion. Urgency to understand this status rises dramatically during and in the aftermaths of pandemics such as the COVID-19 pandemic. Protective immunity is thought to be elicited upon exposure to pathogens such as the SARS-coronavirus 2 (SARS-CoV-2) and recovery from COVID-19. Antibodies generated by a patient's immune system 'neutralize' the virus either via binding to crucial structures of the S protein receptor binding site (RBD) and hence preventing its docking onto its natural receptor, ACE2, in human cells. Alternatively, they hinder the virus later in its infection cycle by blocking penetration and/or uncoating. The standard analytical tool to determine a patient's antibody titer are ELISA or lateral flow assay (LFA) based assay formats which allow for the quantification of binding antibodies. However, such assays do not provide functional insight into the level of virus neutralization.

More advanced tools assessing the neutralizing activity of the patients' antisera are based either on lentiviral or vesicular stomatitis virus particles which are pseudotyped with the coronavirus S protein to acquire infectivity of a real virus in various cell culture models [1]. These investigate the ability of a patient's serum to render a virus non-infective. Specifically, active virus particles or pseudotypes are added to a patient's serum sample. Subsequently, such mixture is added to a susceptible cell culture or indicator cells. In case the antibodies in the serum are able to effectively neutralize the virus or the pseudotyped particles, such virus is not able to infect the cells and multiply, or such particles are not able to elicit reporter gene expression, respectively.

These cell culture tests are slow (16 h), expensive and can only be performed in highly specialized biosafety level 2 (pseudotypes) or 3 (life SARS-CoV-2) labs. To date, no other testing strategy provides similarly reliable results. A mere quantification of anti-virus binding antibodies in patients' serum via much simpler testing platforms (e.g. ELISA, LFA) is not sufficient, as only a minority of binding antibodies are able to neutralize pathogens and/or neutralize infection of susceptible target cells. Thus, high antibody titers may be misleading and do not provide functional information regarding the level of virus neutralization.

Currently, in the COVID-19 pandemic, many labs across the globe try to determine, whether such antibody binding determinations can be correlated to an antibody neutralization titer. However, experience from a myriad of viral infectious diseases makes this highly questionable. Accordingly, there is the need for enhanced assays to detect the functional immune status of a patient, for example by determining whether pathogen-neutralizing molecules are present in a sample of the patient or not.

EP 0 301 333 A2 relates to liposome-based assays for detecting analytes, however, it does not relate to assays for testing neutralizing capacity e.g. the presence of pathogen-neutralizing molecules. Thus, there is the need for neutralization assays which allow for testing the neutralization capacity of a patient with respect to a particular pathogen, such as SARS-CoV-2. Furthermore, there is a need for means that enable the detection of pathogen-neutralizing molecules and/or enable the determination whether a patient is immune to the disease or not. There is also the need to provide means that allow to provide reliable and effective tests for the presence/absence of pathogen-neutralizing molecules, for example methods of detecting pathogen-neutralizing molecules. Moreover, there is a need for kits and POC devices that allow to test whether a pathogen-neutralizing molecule is present or not, for example for determining whether a patient is immune to a pathogen or not. Furthermore, there is a need for means that enable to test whether a patient is immune to pathogens such as SARS-CoV-2.

### SUMMARY OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In a first aspect, the present invention relates to a particle collection for detecting a pathogen-neutralizing molecule, preferably antibody, comprising
- a pathogen-mimicking particle, preferably a non-infectious modified pathogen, a pathogen-like particle such as a virus-like particle (VLP), a fusion protein, and/or a nanoparticle selected from silica nanoparticles, polymeric nanoparticles, e.g. PLA nanoparticles, organic nanoparticles, and inorganic nanoparticles, wherein said pathogen-mimicking particle comprises at least one biomarker, preferably surface biomarker, of said pathogen;
- a pathogen-targeting particle, preferably a liposome, wherein said pathogen-targeting particle comprises
   at least one pathogen-targeting molecule, wherein said pathogen-targeting molecule is capable of binding, preferably specifically binding, to said biomarker of said pathogen; and
   a marker;
- optionally, a complement activating agent,
   wherein, preferably, said complement activating agent recognizes and/or binds to said pathogen-mimicking particle, and/or
   said complement activating agent is incorporated in said pathogen-mimicking particle.

In one embodiment, said at least one biomarker of said pathogen-mimicking particle is presented on a surface, preferably outer surface, of said pathogen-mimicking particle and/or is integrated in said pathogen-mimicking particle,
wherein, optionally, said biomarker is associated with said pathogen-mimicking particle via a transmembrane domain of said biomarker, a linker, a GPI anchor, a PEG, an enzymatic linkage such as a sortase linkage, a homo- or heterobifunctional crosslinker, or natural or non-natural aminoacid(s).

In one embodiment, said biomarker is a SARS-CoV2 biomarker, preferably any of a S (spike) protein, E (envelope) protein, M (membrane) protein, N (nucleocapsid) protein, an HIV envelope biomarker, influenza hemagglutinin, influenza neuraminidase, influenza M protein, an Ebola biomarker, a Marburg virus glycoprotein, a Lassa virus glycoprotein, a Herpes virus biomarker, a bacterial surface biomarker, or a fragment thereof, more preferably a SARS-CoV-2-S protein or a fragment thereof such as the receptor-binding domain thereof; and/or wherein said pathogen-mimicking particle is a SARS-CoV-2-like particle.

In one embodiment, said pathogen-targeting particle comprises a liposome; and/or said pathogen-targeting particle, preferably liposome, is biotinylated, PEGylated, streptavidinylated, anionic, cationic, zwitterionic, coated with other stabilizing molecules such as proteins or polymers, or functionalized with a coupling group such as NH2, COOH, OH, NTA, short peptides, polysaccharides, or short nucleic acid strands; and/or said pathogen-targeting particle has an average size in the range of from 1 nm to 600 nm, preferably 50 nm to 300 nm, more preferably 75 nm to 250 nm, e.g. 150 nm.

In one embodiment, said pathogen-targeting molecule is any of a receptor, ligand, enzyme, and a fragment thereof, preferably transmembrane protease serine 2 (TMPRSS2), ACE2, CD4, CCR5, CXCR4, or a fragment thereof, more preferably ACE2 or a fragment thereof.

In one embodiment, said pathogen-targeting particle comprises a marker selected from a fluorescent marker, preferably sulforhodamine B or carboxyfluorescein, an electrochemical marker, preferably potassium hexaferricyanide or hexaferrocyanide, or ruthenium hexamine, a colorimetric marker, preferably sulforhodamine B, a chemiluminescent marker, preferably m-COOH luminol, an electrochemiluminescent marker, preferably mCOOH-luminol or ruthenium bipyridyl, a bioluminescent marker such as GFP, or an enzyme substrate such as glucose, lactate or ATP, an enzyme such as glucose oxidase, alkaline phosphatase, or peroxidase, or DNA molecules,wherein, optionally, said marker is contained inside the pathogen-targeting particle, preferably in an inner space of said pathogen-targeting particle.

In one embodiment, said marker is contained inside the pathogen-targeting particle, preferably in an inner space of said pathogen-targeting particle.

In one embodiment, said complement activating agent comprises any of an antibody, a peptide, e.g. binding peptide, a protein, e.g. Protein A or G, streptavidin, biotin, a lectin, a carbohydrate, cholesterol, PEG, a nucleic acid, an aptamer, a complement component, e.g. a C3b-fragment, a microbial component, e.g. LPS, a component of an apoptotic cell, a pentraxin, and fragments, combinations, or multimers thereof;
wherein, optionally, said complement activating agent further comprises a complement activating moiety,
wherein, preferably, said complement activating moiety is selected from LPS, a Fc domain or a Fc-multimer, a Fc CH3 domain or a CH3-multimer, a carbohydrate, and a complement component, e.g. a C3b-fragment.

In one embodiment, said complement activating agent recognizes and/or binds to said at least one biomarker of said pathogen-mimicking particle; and/or
said complement activating agent recognizes and/or binds to a target, e.g. a peptide, on said pathogen-mimicking particle other than the at least one biomarker; and/or
said complement activating agent is incorporated in said pathogen-mimicking particle such that said complement activating agent and/or a complement activating moiety comprised by said complement activating agent is/are presented on a surface of said pathogen-mimicking particle.

In a further aspect, the present invention relates to a composition comprising a particle collection, as defined above, wherein said composition comprises any of a pathogen-mimicking particle, a pathogen-targeting particle, and a complement activating agent, preferably comprises a pathogen-mimicking particle and a pathogen-targeting particle, and/or a pathogen-mimicking particle, a pathogen-targeting particle, and a complement activating agent.

In this aspect, said particle collection, said pathogen-mimicking particle, said pathogen-targeting particle, and said complement activating agent are as defined above.

In a further aspect, the present invention relates to a method of detecting a pathogen-neutralizing molecule, preferably antibody, using a particle collection, as defined above, or a composition, as defined above, preferably an *in vitro* method of detecting a pathogen-neutralizing antibody of a patient to a pathogen or a method for screening pathogen-targeting compound(s), comprising the steps:
i) contacting a sample with said pathogen-mimicking particle,
   wherein, optionally, said sample is a sample obtained from a patient, preferably a serum sample, or said sample is a target compound to be tested or compound library to be screened,
ii) optionally, incubating said sample with said pathogen-mimicking particle, e.g. for 0 to 120 minutes at a temperature of from 4 to 40 °C,
iii) adding said pathogen-targeting particle comprising a marker thereto, preferably a liposome,
iv) optionally, incubation, e.g. for 0 to 120 minutes at a temperature of from 15 to 40 °C, preferably 20 to 37 °C, such as 22 °C,
v) optionally, adding said complement activating agent and/or standardized blood components thereto,
vi) optionally, incubation, e.g. for 10 to 120 minutes at a temperature of from 20 to 40 °C, preferably 37 °C,
vii) detecting a signal of said marker;
wherein, optionally, said method comprises a step of immobilizing said pathogen-targeting particle.

In one embodiment, said detecting is performed using any of colorimetric, fluorescent, chemiluminescent, electrochemiluminescent, electrochemical, bioluminescent, and/or visual detection, optionally detection using a cell phone; and/or
said marker is selected from a fluorescent marker, preferably sulforhodamine B or carboxyfluorescein, an electrochemical marker, preferably potassium hexaferricyanide or hexaferrocyanide, or ruthenium hexamine, a colorimetric marker, preferably sulforhodamine B, a chemiluminescent marker, preferably m-COOH luminol, an electrochemiluminescent marker, preferably mCOOH-luminol or ruthenium bipyridyl, a bioluminescent marker such as GFP, or an enzyme substrate such as glucose, lactate or ATP, an enzyme such as glucose oxidase, alkaline phosphatase, or peroxidase, or DNA molecules.

In one embodiment, said method further comprises determining a presence of a pathogen-neutralizing molecule, preferably antibody, if a signal detected in step iv) is increased compared to a reference signal and/or reference value.

In this aspect, said particle collection, said pathogen-mimicking particle, said pathogen-targeting particle, said pathogen-targeting molecule, said complement activating agent, said marker, and said composition are as defined above.

In a further aspect, the present invention relates to a kit for detecting a pathogen-neutralizing molecule, preferably antibody, comprising a particle collection, as defined above, or a composition, as defined above, further comprising any of
- an auxiliary agent, preferably any of a buffer, a solvent, standardized blood components, e.g. standardized human blood components, a standardized serum, inactivated serum, an indicator dye, and a detergent;
- optionally, a test strip;
- optionally, an electrode;
- optionally, a microtiterplate;
- optionally, instructions for determining a presence of a pathogen-neutralizing molecule, preferably antibody, if a signal of a marker is increased compared to a reference signal and/or reference value.

In this aspect, said particle collection and said composition are as defined above.

In a further aspect, the present invention relates to a point-of-care device, preferably an electrochemical and/or lateral-flow assay point-of-care device, comprising
- an inlet for receiving at least one sample, preferably a serum sample;
- a contacting unit for contacting said sample(s) with any of a pathogen-mimicking particle, a pathogen-targeting particle comprising a marker, a complement activating agent, and combinations thereof, preferably for contacting said sample(s) with a particle collection, as defined above, or a composition, as defined above;
- optionally, a test line with an immobilized and/or adsorbed recognition element, for example streptavidin, polystreptavidin, antibodies against digoxygenin or FITC, biotinylated BSA, or an affinity recognition element such as avidin, Protein A or G, a polymer, a lectin, a protein or polymer labeled with haptens, charged molecules, NTA, or DNA molecules;
- optionally, a control line containing an immobilized and/or adsorbed control recognition element, for example streptavidin, polystreptavidin, antibodies against digoxygenin or FITC, biotinylated BSA, or an affinity recognition element such as avidin, Protein A or G, a polymer, a lectin, a protein or polymer labeled with haptens, charged molecules, NTA, or DNA molecules;
- optionally, a pH indicator, e.g. a pH indicator dye, for example impregnated in a sample pad or conjugate pad or waste pad;
- optionally, a waste pad impregnated with a buffer for providing a color change of a pH indicator once the pH indicator reaches the waste pad;
- a detecting unit for detecting a signal of said marker, preferably a detecting unit for colorimetric, fluorescent, chemiluminescent, electrochemiluminescent, electrochemical, bioluminescent, and/or visual detection, optionally detection using a cell phone;
- optionally, an output unit for outputting a result obtained from said detecting.

In one embodiment, the point-of-care device, as defined above, is for use in combination with a kit, as defined above, preferably for use in a method, as defined above.

In this aspect, said sample, said particle collection, said pathogen-mimicking particle, said pathogen-targeting particle, said marker, said complement activating agent, said composition, said contacting, said detecting, said kit, and said method are as defined above.

In a further aspect, the present invention relates to a use of a particle collection, as defined above, a composition, as defined above, a kit, as defined above, and/or a point-of-care device, as defined above, in a method of detecting a pathogen-neutralizing molecule, preferably antibody, preferably a method as defined above.

In a further aspect, the present invention relates to a use of a particle collection, as defined above, a composition, as defined above, a kit, as defined above, a point-of-care device, as defined above, and/or a method, as defined above, for determining whether a pathogen-neutralizing molecule is present in a sample.

In a further aspect, the present invention relates to a method of diagnosing immunity of a patient, comprising using a particle collection, as defined above, a composition, as defined above, a kit, as defined above, a point-of-care device, as defined above, and/or a method, as defined above, for determining whether a pathogen-neutralizing molecule is present in a sample of said patient, preferably an *in vitro* method of diagnosing.

In a further aspect, the present invention relates to a method of screening pathogen-targeting compound(s) and/or screening a library comprising pathogen-neutralizing molecule candidates, comprising using a particle collection, as defined above, a composition, as defined above, a kit, as defined above, and/or a point-of-care device, as defined above, for determining whether a pathogen-neutralizing molecule is present in a sample comprising at least one target compound and/or pathogen-neutralizing molecule candidate.

### DETAILED DESCRIPTION

Herein presented is a novel assay format based on signaling molecule entrapping pathogen-targeting particles, such as liposomes. The binding of an analyte to the pathogen-targeting particle, e.g. liposome, will trigger lysis of the pathogen-targeting particle through a serum-induced and complement related reaction that leads to the release of the entrapped marker molecules from the pathogen-targeting particle. This concept can be exploited in homogeneous assays, in which no separation from intact and lysed pathogen-targeting particle, preferably liposomes, is needed, as only the released marker molecules are detectable. This works for example by using fluorescent dyes that self-quench at high concentrations inside the pathogen-targeting particle, or enzymes or their substrates.

Alternatively, pathogen-targeting particles, e.g. liposomes, are bound to surfaces so that the free marker molecules can be washed away. This strategy is feasible with any marker molecule (fluorescent, colorimetric, chemiluminescent, etc.) and in a variety of assay formats including microtiter plate, lateral flow assay and transducer-based strategies. Here, the inventors demonstrate the generation of liposomes that are not lysed when in contact with human serum (stealth liposomes), but that can be deliberately lysed in human serum by the addition of trigger molecules such as lipopolysaccharides (LPS) or antibodies. This lysis does not occur in inactivated serum. In contrast, the inventors also show that cationic liposomes are lysed through serum protein interactions that is not related to the complement system but will take place in active or inactivated serum. This may also be a lysis strategy in this envisioned assay. Finally, the inventors demonstrate that proteins can be coupled to liposomes, such as streptavidin and ACE2. The inventors also demonstrate that stealth liposomes remain intact when incubated with virus-like particles (VLP). In one embodiment, the particle collection, composition, method, kit, point-of-care device, and use of the invention are means for neutralization assays. In one embodiment, a particle collection, composition, kit, and/or point-of-care device can be used for performing a neutralizing assay, preferably using a method of the invention. In one embodiment, a method of the invention is a neutralization assay.

The term "particle collection", as used herein, relates to a collection e.g. group of molecules and/or particles. In one embodiment, the terms "particle collection" and "particle assembly" can be used interchangeably. In one embodiment, a particle collection comprises at least two types of particles, namely at least a pathogen-mimicking particle and a pathogen-targeting particle, and optionally further a complement activating agent. In one embodiment, a composition comprises at least two components of a particle collection, namely at least two of a pathogen-mimicking particle, a pathogen-targeting particle, and a complement activating agent. In one embodiment, the particle collection is a particle assembly. In one embodiment, said particle collection further comprise(s) a pH indicator, optionally selected from phenolphthalein, bromothymol blue, and litmus. In one embodiment, said particle collection further comprises a chromogenic dye such as a food coloring or sulforhodamine B. In one embodiment, a pH indicator that undergoes a color change from acidic to neutral/slightly alkaline is suitable to be used in and/or for a particle collection, composition, method, kit, POC device of the invention. In one embodiment, a pH indicator acts as a flow-control for the lateral flow strip, e.g. only when sample flow from sample pad to waste pad is correct, a color shift will be visible in the waste pad.

The term "pathogen", as used herein, relates to any organism that can cause a disease. In one embodiment, a pathogen is any organism that can cause a disease, such as a virus, bacterium, protozoan, prion, viroid, or fungus. In one embodiment, the pathogen is a coronavirus causing COVID-19 and/or is severe acute respiratory syndrome coronavirus 2, or is HIV.

The term "pathogen-neutralizing molecule", as used herein, relates to a molecule, such as an antibody, that neutralizes a pathogen. In one embodiment, "neutralizing" refers to making a pathogen noninfectious and/or to reducing infectivity of a pathogen, for example by preventing a pathogen, such as a virus, to enter a cell, such as a cell of a patient and/or a healthy individual. In one embodiment, a pathogen-neutralizing molecule e.g. a neutralizing antibody is a molecule e.g. an antibody that defends a cell from a pathogen or infectious particle by neutralizing any effect it has biologically. In one embodiment, pathogen-neutralizing molecules, e.g. neutralizing antibodies, are part of the humoral response of the adaptive immune system against viruses, intracellular bacteria and microbial toxin. In one embodiment, the presence of a pathogen-neutralizing molecule in a subject makes the subject immune to said pathogen. In one embodiment, if a subject, such as a patient, has pathogen-neutralizing molecules, the subject is immune to said pathogen. In one embodiment, a pathogen-neutralizing molecule causes immunity to said pathogen. In one embodiment, immunity due to pathogen-neutralizing molecules e.g. neutralizing antibodies is also known as sterilizing immunity, as the immune system eliminates the infectious particle before any infection takes place. In one embodiment, a method of detecting a pathogen-neutralizing molecule is an *in vitro* method of detecting a pathogen-neutralizing molecule, such as an antibody, of the patient, wherein said pathogen-neutralizing molecule is a molecule produced by the immune system of the patient to eliminate said pathogen. In one embodiment, a pathogen-neutralizing molecule of a patient is a molecule produced by the immune system of the patient to eliminate said pathogen. In one embodiment, a method of detecting a pathogen-neutralizing molecule is a method for screening pathogen-targeting compound(s), wherein said pathogen-targeting compound is a molecule capable of neutralizing a pathogen and/or a molecule capable of reducing infectivity of said pathogen and/or a molecule capable of binding to said pathogen, e.g. a drug. In one embodiment, a target compound is a molecule which is a pathogen-targeting compound candidate and/or a pathogen-neutralizing molecule candidate, e.g. an antibody, antibody fragment, antigen-binding fragment, or aptamer to be tested. In one embodiment, a compound library is a library of target compounds, i.e. a library of pathogen-targeting compound candidates and/or pathogen-neutralizing molecule candidates. In one embodiment, a pathogen-neutralizing molecule is a pathogen-neutralizing molecule, e.g. pathogen-neutralizing antibody, of a patient, and/or is a pathogen-targeting compound which is a drug to be used for neutralizing said pathogen.

The term "antibody", as used herein, relates to a molecule, such as an immunoglobulin (Ig) which is used by the immune system to identify and neutralize pathogens such as pathogenic bacteria and viruses. In one embodiment, when referring to a pathogen-neutralizing molecule being an antibody, such antibody is a neutralizing antibody defending a cell from a pathogen or infectious particle by neutralizing any effect it has biologically, e.g. preventing cell entry of a virus. In one embodiment, neutralizing antibodies inhibit the infectivity of a pathogen by binding to the pathogen and/or blocking the molecules needed for cell entry. For example, in case of a virus infection, neutralizing antibodies can bind to glycoproteins of enveloped viruses or capsid proteins of non-enveloped viruses. Furthermore, neutralizing antibodies can act by preventing particles from undergoing structural changes needed for cell entry. For example, neutralizing antibodies may prevent conformational changes of viral proteins that mediate the membrane fusion needed for entry into the host cell. Neutralizing antibodies can also be used for neutralizing the toxic effects of bacterial toxins. In one embodiment, the terms "pathogen-neutralizing antibody" and "neutralizing antibody" are used interchangeably. There are non-neutralizing antibodies and neutralizing antibodies. "Non-neutralizing antibodies", also referred to as "binding antibodies", bind specifically to the pathogen, but do not interfere with the infectivity of the pathogen. In one embodiment, non-neutralizing antibodies can be used to flag the pathogen and/or pathogen-mimicking particle for immune cells and/or the complement system. In one embodiment, a compliment activating agent is a non-neutralizing antibody.

The term "pathogen-mimicking particle" or abbreviated "PMP", as used herein, relates to a particle that mimics at least one feature of a pathogen, e.g. a particle comprising a pathogen surface molecule, such as a surface protein or fragment thereof. In one embodiment, a pathogen-mimicking particle is a noninfectious replacement for a pathogen in a test system, such as an assay. In one embodiment, a pathogen-mimicking particle comprises one or more surface structures and/or surface molecules of said pathogen, e.g. a peplomer and/or a spike protein of a virus. In one embodiment, the pathogen-mimicking particle is a SARS-CoV-2-mimicking particle comprising at least the spike (S) protein of SARS-CoV-2. In one embodiment, such pathogen-mimicking particle, e.g. SARS-CoV-2-mimicking particle, may be present in the form of a virus-like particle (VLP), a fusion protein comprising a S protein, and/or a nanoparticle comprising a S protein. In one embodiment, a pathogen-mimicking particle comprises at least one biomarker, wherein said biomarker is a molecule, e.g. surface molecule, of said pathogen, wherein said molecule is typically targeted by neutralizing antibodies against said pathogen. In one embodiment, said biomarker is a surface molecule of a pathogen, wherein said surface molecule is bound by an anti-pathogen neutralizing antibody. In one embodiment, the term "molecule", preferably in the context of biomarkers, refers to any molecule, e.g. cell-surface presented molecule, such as a protein, peptide, cluster-of-differentiation molecule, receptor, ligand, lipid, ion channel, sugar, and glycopeptide. In one embodiment, a pathogen-mimicking particle is a SARS-CoV-2-like particle, preferably comprising the viral spike protein (S) or a S variant, and/or comprising the viral proteins N, M, E, and S or a S variant, optionally further comprising a transmembrane domain or a GPI anchor, and/or is a fusion protein comprising the viral spike protein (S) or a S variant. Such "variant" of a molecule, e.g. protein, is a biologically active derivative or fragment of the molecule, wherein "biologically active" means that it has a biological function e.g. binding function of the molecule, for example such variant has a receptor binding domain of said molecule. In one embodiment, the pathogen-mimicking particle is a fusion protein which comprises a receptor binding domain or envelope protein, or a multimer thereof, optionally fused to a complement trigger, such as a complement activating agent. In one embodiment, said fusion protein comprises ferritin fused to a receptor binding domain or envelope protein. In one embodiment, the fusion protein comprises or consists of a multimer, e.g. a trimer or hexamer, of a receptor binding domain or envelope protein, e.g. of a SARS-CoV-2 or SARS-CoV-1 receptor binding domain. In one embodiment, the pathogen-mimicking particle is a fusion protein of a SARS-CoV-2 or SARS-CoV-1 protein, e.g. receptor binding domain, and a complement activating agent. For example, a S protein or receptor binding domain (RBD), or a trimer of a S protein or RBD, e.g. a trimer formed via a C-terminal GCN-trimerization domain, could be fused to a complement activating agent, e.g. fused via the C-terminus. In one embodiment, when referring to a "virus particle", a PMP is meant.
The term "non-infectious modified pathogen", as used herein, relates to a pathogen, e.g. a previously infectious pathogen that has been modified to be noninfectious. In one embodiment, a non-infectious modified pathogen is a previously pathogenic particle and/or pathogen that has/have been rendered noninfectious, for example by physical and/or chemical modification, e.g. via heat, irradiation, chemicals, sterilization, or high-pressure. In one embodiment, a non-infectious modified pathogen is a pathogen modified by chemicals, heat, pressure, and/or irradiation to become a non-infectious modified pathogen.

In one embodiment, the term "pathogen-like particle" relates to a particle which comprises at least one feature of at least one pathogen, for example at least one surface molecule, e.g. surface protein, or fragment thereof of at least one particular pathogen. In one embodiment, a pathogen-like particle comprises one or several pathogen biomarkers of one or several pathogens. In one embodiment, a pathogen-like particle comprises a SARS-CoV2 protein, SARS-CoV1 protein, an HIV biomarker, or any combination thereof. In one embodiment, a pathogen-like particle is genetically engineered. In one embodiment, a pathogen like particle may comprise features such as proteins of more than one pathogen, such as a pathogen-like particle comprising a lentiviral group specific antigen such as e.g. HIV Gag and a CoV2 S protein. In one embodiment, the pathogen-like particle is a SARS-CoV-2-like particle, preferably comprising the viral spike protein (S) or a S variant, and/or comprising the viral proteins N, M, E, and S or a S variant, optionally further comprising a transmembrane domain or a GPI anchor. The term "virus-like particle", as used herein, relates to a particle that comprises one or more features of a virus, preferably comprises at least one surface molecule e.g. surface protein of a virus. In one embodiment, a virus-like particle is not infectious. In one embodiment, when referring to "features" in the context of features of a pathogen, such features are preferably surface molecules, such as surface proteins. In one embodiment, a virus-like particle comprises those molecules, preferably proteins, of a virus sufficient to form a particle, e.g. comprises an S protein, and/or comprises any of an N protein, M protein, E protein, and S protein or combinations thereof. In one embodiment, a pathogen-like particle, preferably virus-like particle, is a virus-like particle or similar to a virus-like particle as described in [4].

The term "fusion protein", as used herein, relates to proteins in which at least two proteins and/or peptides are joined. In one embodiment, a fusion protein is a protein created through the joining of two or more genes that originally coded for separate proteins (fusion gene), and translation of such fusion gene. In one embodiment, a fusion protein has functional properties derived from each of the joined proteins. In one embodiment, a pathogen-mimicking particle is a fusion protein of at least two proteins of said pathogen, and/or is a fusion protein of at least one protein of said pathogen and a complement activating agent, preferably a protein-comprising complement activating agent. In one embodiment, the terms "protein" and "peptide" are used interchangeably. In one embodiment, a fusion protein is a protein dimer, trimer or multimer. In one embodiment, a fusion protein comprises a protein, such as ferritin, and further comprises a pathogen biomarker, e.g. a receptor binding domain of a pathogen.

In one embodiment, the term "nanoparticle" relates to any nanoparticle known to the person skilled in the art, e.g. silica nanoparticles, polymeric nanoparticles, organic nanoparticles, and inorganic nanoparticles, for example any of PLA nanoparticles, CaP nanoparticles, PLG nanoparticles, liposomes, gold nanoparticles, and carbon dots. In one embodiment, said nanoparticle comprise at least one biomarker, for example is labeled with said at least one biomarker. In one embodiment, a biomarker is attached to and/or bound to said nanoparticle via a linker, for example via PEG, a GPI anchor, a "click" chemistry linker, or any other linker known to a person skilled in the art.

The term "biomarker", as used herein, relates to a molecule of a pathogen, preferably a molecule presented on the surface of a pathogen. In one embodiment, a biomarker is a molecule of a pathogen bound by a neutralizing antibody. In one embodiment, a biomarker is a molecule of a pathogen selected and/or configured to be bound by a neutralizing antibody.

In one embodiment, a biomarker is a target of a neutralizing antibody. In one embodiment, a biomarker is a molecule, e.g. protein, of a pathogen involved in infectivity of said pathogen. In one embodiment, a biomarker is a molecule involved in binding of a pathogen to a target cell e.g. a cell of the patient. In one embodiment, a biomarker is a surface molecule, such as a surface protein, surface peptide, surface glycoprotein, surface lipid, surface-presented ligand, surface-presented receptor, surface-presented enzyme, surface-presented enzyme substrate of said pathogen. In one embodiment, the terms "surface molecule" and a molecule being "surface-presented" mean that at least a portion of said molecule is presented on and/or bound to the surface of a pathogen and/or particle e.g. pathogen-mimicking particle. In one embodiment, said biomarker is a SARS-CoV2 biomarker, preferably any of a S (spike) protein, E (envelope) protein, M (membrane) protein, N (nucleocapsid) protein, an HIV envelope biomarker, influenza hemagglutinin, influenza neuraminidase, influenza M protein, an Ebola biomarker, a Marburg virus glycoprotein, a Lassa virus glycoprotein, a Herpes virus biomarker, a bacterial surface biomarker, or a fragment thereof, more preferably a SARS-CoV-2-S protein or a fragment thereof such as the receptor-binding domain thereof. In one embodiment, when referring to a "pathogen", a "pathogen-mimicking particle" is also meant. In one embodiment, the term "is associated with" is used interchangeably with "is bound to" and/or "is coupled to". In one embodiment, a biomarker is coupled to a pathogen-mimicking particle via streptavidin-biotin interaction or a pathogen targeting molecule is coupled to a pathogen-targeting particle via streptavidin-biotin interaction.

The term "pathogen-targeting particle" or abbreviated "PTP", as used herein, relates to a particle that is configured to bind to a pathogen and/or pathogen-mimicking particle. In one embodiment, such pathogen-targeting particle comprises a pathogen-targeting molecule which is capable of and/or configured to bind to a pathogen, preferably to a biomarker of a pathogen-mimicking particle. In one embodiment, the pathogen-targeting particle binds, preferably specifically binds, to the at least one biomarker of the pathogen-mimicking particle. In one embodiment, the pathogen-targeting particle binds, preferably specifically binds, to the pathogen-mimicking particle. In one embodiment, the pathogen-targeting particle binds, preferably specifically binds, to the pathogen-mimicking particle, preferably to the at least one biomarker of the pathogen-mimicking particle, via said pathogen-targeting molecule of said pathogen-targeting particle, for example via ligand-receptor binding, enzyme-substrate binding, or antigen-antibody binding. In one embodiment, the pathogen-targeting particle is any particle known to a person skilled in the art, preferably particle that can be lysed by the complement system, e.g. a nanoparticle or liposome, preferably a liposome. In one embodiment, said pathogen-targeting particle, e.g. liposome, is PEGylated, wherein, optionally, a PEG molecule of the PEGylation has a functional group for modification of said pathogen-targeting particle, preferably an amino group, carboxy group, azide group, or biotin. The term "liposome", as used herein, relates to a lipid-membrane based nano- or microvesicle having a lipid bilayer. Liposomes are phospholipid bilayer nanovesicles that can be synthesized with full control of lipid composition, surface chemistry, and size. In one embodiment, a liposome has a long-term stability >4 years at 4 °C. In one embodiment, a liposome comprises polyethylene glycol (PEG) and/or cholesterol.

The term "pathogen-targeting molecule", as used herein, relates to a molecule capable of binding, preferably specifically binding, to said biomarker of said pathogen and/or to said at least one biomarker comprised by said pathogen-mimicking particle. In one embodiment, the combination of biomarker and pathogen-targeting molecule provide for a specific binding between the pathogen-mimicking particle and the pathogen-targeting particle. In one embodiment, the at least one biomarker of the pathogen-mimicking particle and the pathogen-targeting molecule specifically bind to each other and/or interact with each other. In one embodiment, the pathogen-mimicking particle and the pathogen-targeting particle specifically bind to each other and/or interact with each other via the at least one biomarker of the pathogen-mimicking particle and the pathogen-targeting molecule of the pathogen-targeting particle. In one embodiment, such specific interaction and/or binding is prevented and/or decreased if a neutralizing antibody binds to and/or is bound to said biomarker. In one embodiment, an absence of binding between the pathogen-mimicking particle and the pathogen-targeting particle indicates the presence of a neutralizing antibody binding to the biomarker. In one embodiment, said pathogen-targeting molecule is any of a receptor, ligand, enzyme, and a fragment thereof, preferably transmembrane protease serine 2 (TMPRSS2), ACE2, CD4, CCR5, CXCR4, or a fragment thereof, more preferably ACE2 or a fragment thereof. In one embodiment, ACE2 on a cell is bound by SARS-CoV-2, e.g a spike protein of SARS-CoV-2. In one embodiment, if the pathogen is SARS-CoV-2, the pathogen-targeting molecule is a molecule targeting SARS-CoV-2, preferably targeting a spike protein of SARS-CoV-2, for example is ACE2. In one embodiment, CXCR4 on a cell is bound by HIV. In one embodiment, if the pathogen is HIV, the pathogen-targeting molecule is a molecule targeting HIV, for example is CXCR4.

The term "marker", as used herein, relates to a molecule that can be detected, preferably quantitatively analyzed, via any detection method and/or analysis method known to a person skilled in the art, e.g. via chromatography, electrophoresis, microscopy, photometry, spectroscopy, such as atomic absorption spectroscopy, ultraviolet-visible spectroscopy, x-ray spectroscopy, fluorescence spectroscopy, infrared spectroscopy, Raman spectroscopy, nuclear magnetic resonance spectroscopy, photoemission spectroscopy, mass spectrometry, electrochemical analysis, calorimetry, sequencing, precipitation, or extraction. In one embodiment, a marker is selected from 1) a fluorescent marker, preferably sulforhodamine B or carboxyfluorescein, 2) an electrochemical marker, preferably potassium hexaferricyanide or hexaferrocyanide, or ruthenium hexamine, 3) a colorimetric marker, preferably sulforhodamine B, 4) a chemiluminescent marker, preferably m-COOH luminol, 5) an electrochemiluminescent marker, preferably mCOOH-luminol or ruthenium bipyridyl, 6) a bioluminescent marker such as GFP, 7) an enzyme substrate such as glucose, lactate or ATP, 8) an enzyme such as glucose oxidase, alkaline phosphatase, or peroxidase, or 9) DNA molecules. In one embodiment, said marker is contained inside the pathogen-targeting particle, preferably in an inner space of said pathogen-targeting particle. In one embodiment, said pathogen-targeting particle comprises a compartment and/or a reservoir containing said marker. In one embodiment, said marker provides a signal only when released from said pathogen-targeting particle. Alternatively, said marker provides a signal only when contained inside the pathogen-targeting particle, and said signal dissipates when the marker is released form the pathogen-targeting particle. The skilled person understands that such marker is selected depending on the assay format, such as based on whether the pathogen-targeting particles used in an assay format are immobilized or not. In one embodiment, if a marker is a DNA molecule, such DNA molecule can be detected using PCR, LAMP, or any other method known to a person skilled in the art. In one embodiment, a particle is immobilized on a substrate and/or surface by any means known to a person of skill in the art, e.g. streptavidin-biotin interaction.

The term "complement activating agent", as used herein, relates to an agent e.g. molecule that activates the complement system, an agent e.g. molecule that activates at least one component of the complement system, and/or an agent e.g. molecule that triggers lysis of a pathogen-targeting particle. In a preferred embodiment, a complement activating agent is an agent that activates the complement system and/or activates at least one component, i.e. one or more components, of the complement system. In one embodiment, if a complement activating agent is an agent that triggers lysis of a pathogen-targeting particle, such agent is preferably an agent comprised by a sample of a patient, preferably blood sample, e.g. serum or plasma sample. In one embodiment, a complement activating agent, e.g. an agent that triggers lysis of a pathogen-targeting particle, specifically triggers lysis of a pathogen-targeting particle. In one embodiment, "specifically" in the context of triggering lysis means that such lysis of a PTP occurs only if said PTP is present in the form of a PTP-PMP complex and/or if said PTP is bound to a PMP. In one embodiment, the complement activating agent directly activates the complement system, or the complement activating agent activates the complement system via a complement activating moiety comprised by said complement activating agent. In one embodiment, the complement activating agent comprises any molecule selected from an antibody, a peptide, e.g. binding peptide, a protein, e.g. Protein A or G, streptavidin, biotin, a lectin, a carbohydrate, cholesterol, PEG, a nucleic acid, an aptamer, a complement component, e.g. a C3b-fragment, a microbial component, e.g. LPS, a component of an apoptotic cell, a pentraxin, and fragments, combinations, or multimers thereof; wherein, optionally, said complement activating agent further comprises a complement activating moiety such as LPS, a Fc domain or a Fc-multimer, a Fc CH3 domain or a CH3-multimer, a carbohydrate, and a complement component, e.g. a C3b-fragment. In one embodiment, if said complement activating agent alone is not sufficient to trigger a complement response, e.g. streptavidin or biotin, such complement activating agent may be modified by coupling a complement activating moiety thereto. In one embodiment, said complement activating agent recognizes and/or binds to said at least one biomarker of said pathogen-mimicking particle; and/or said complement activating agent recognizes and/or binds to a target molecule, e.g. peptide, on said pathogen-mimicking particle other than the at least one biomarker (e.g. a generic protein/peptide selected from FHR-1, ARMS2, FoxP, and BBS6); and/or said complement activating agent is incorporated in said pathogen-mimicking particle such that said complement activating agent and/or a complement activating moiety comprised by said complement activating agent is/are presented on a surface of said pathogen-mimicking particle. In one embodiment, the pathogen-mimicking particle comprises and/or is bound by the complement activating agent. In one embodiment, a pathogen-mimicking particle incorporates a complement activating agent, preferably presents a complement activating agent on a surface of the pathogen-mimicking particle. In one embodiment, a complement activating agent recognizes and/or binds to said at least one biomarker of said pathogen-mimicking particle, wherein such recognizing and/or binding is preferably a non-neutralizing binding, i.e. the pathogen-mimicking particle is not neutralized by said binding of the complement activating agent to the pathogen-mimicking particle.

In one embodiment, said complement activating agent is configured to bind to a PMP-PTP-complex, or and/or is configured to bind to a PMP, for example via said biomarker or via a molecule, such as a peptide, presented on a surface of said PMP, and/or is configured to be incorporated in, preferably presented by, said PMP. In one embodiment, said complement activating agent comprises or consists of a non-neutralizing antibody, or fragment thereof, capable of binding to said pathogen-mimicking particle, e.g. a VLP-specific non-neutralizing antibody. In one embodiment, said complement activating agent is tagged with a complement activating moiety. In one embodiment, the terms "recognizing" and "binding" refer to binding in general, wherein "recognizing" may relate to a specific binding and "binding" to a specific or nonspecific binding, and/or the terms are used interchangeably. In one embodiment, if said complement activating agent recognizes and/or binds to said at least one biomarker of said pathogen-mimicking particle, such recognition and/or binding is a non-neutralizing binding.

In one embodiment, the term "method of detecting a pathogen-neutralizing molecule", as used herein, relates to a method of detecting whether a pathogen-neutralizing molecule and/or pathogen-targeting compound is present in a sample, e.g. in a patient sample or in a sample comprising at least one pathogen-targeting compound candidate. A "candidate" is a compound to be tested for a specific function, e.g. the function of binding and/or neutralizing pathogens and/or pathogen-mimicking particles. In one embodiment, a method of detecting a pathogen-neutralizing molecule is a method of testing whether an interaction between a pathogen-mimicking particle and a pathogen-targeting particle is prevented and/or inhibited by a pathogen-neutralizing molecule, e.g. pathogen neutralizing antibody or pathogen-targeting compound. In one embodiment, said method is an *in vitro* method of detecting a pathogen-neutralizing antibody of a patient to a pathogen or a method for screening pathogen-targeting compound(s). In one embodiment, the kit and/or point-of-care device is/are used in a method of the present invention. In one embodiment, the method of the invention is performed using a particle collection, composition, kit and/or point-of-care device of the invention. In one embodiment, a method of detecting a pathogen-neutralizing molecule comprises adding a standardized serum, such as a standardized serum diluted in a buffer. In one embodiment, such standardized serum provides a reference, e.g. a reference signal and/or reference value. In one embodiment, a method of detecting a pathogen-neutralizing molecule comprises adding standardized blood components, e.g. standardized human blood components. In a preferred embodiment, standardized blood components are standardized human blood components. In one embodiment, such standardized blood components, preferably standardized human blood components, provide a reference, e.g. a reference signal and/or reference value. In one embodiment, blood components, e.g. standardized blood components, comprise or consist of serum and/or plasma components. In one embodiment, standardized blood components have a defined composition, e.g. a defined serum composition, defined plasma composition, and/or defined complement composition. In one embodiment, standardized blood components are added in a method of detecting a pathogen-neutralizing molecule, wherein a sample is obtained from a patient having a deficient complement system. In one embodiment, standardized blood components, such as standardized serum, comprise complement components.

The term "sample", as used herein, relates to a sample to be tested, preferably a sample of a patient and/or healthy individual, or a sample comprising at least one pathogen-targeting compound candidate, e.g., a compound library of pathogen-targeting compound candidates. In one embodiment, the term "subject" relates to a patient and/or healthy individual. In one embodiment, said sample is a sample obtained from a patient and/or a sample of a patient, preferably a serum sample, or said sample is a target compound to be tested or compound library to be screened. In one embodiment, said sample of a patient is a serum sample or a diluted serum sample. For example, if the method of the invention is a method of detecting a pathogen-neutralizing antibody of a patient, e.g. an *in vitro* method of detecting a pathogen-neutralizing antibody, said sample is a sample, preferably blood sample, e.g. serum or plasma sample, obtained from said patient. For example, if the method of the invention is a method for screening pathogen-targeting compound(s), said sample comprises or consists of a target compound to be tested or a compound library to be screened.

The term "detecting a signal of a marker", as used herein, comprises direct and/or indirect detection and/or analysis of a marker signal, for example using any of colorimetric, fluorescent, chemiluminescent, electrochemiluminescent, electrochemical, bioluminescent, and/or visual detection, optionally detection using a cell phone. For example, a signal of a marker can be detected via i) direct detection, e.g. of a fluorescent of bioluminescent signal, via ii) previous immobilization of liposomes on a surface and subsequent detection, and/or via iii) previous collecting liposomes from a solution and subsequent detection. In one embodiment, liposomes are immobilized, e.g. via biotin on liposomes and streptavidin on a surface, or via digoxygenin on liposomes and an anti-dig antibody on a surface, or via FITC on liposomes and anti-FITC immobilized on a surface, followed by a washing step, e.g. a washing step after step (iii) of a method of detecting a pathogen-neutralizing molecule, optionally addition of a detergent, and then the signal is detected. In one embodiment, there are at least three different options for the detection:
Scenario 1: direct detection of signal
Scenario 2: immobilization of liposomes on a surface, e.g. in step iii), preferably prior to contacting the liposomes with the patient sample and/or the pathogen-mimicking particle. In this case, a subsequent step comprises the following:
   1. Removal of supernatant
   2. Washing
   3. Addition of liposome lysis agent (e.g. a detergent, an organic solvent, heat) and detection.
Scenario 3: collection of all liposomes from solution (e.g. via magnetic beads, or by addition to a surface to which the liposomes will bind). Washing the collected liposomes, and then detection step 3 of scenario 2.

The term "reference signal and/or reference value", as used herein, relates to a signal and/or a value obtained for a sample that does not comprise a pathogen-neutralizing molecule. In one embodiment, such reference signal and/or reference value is a signal and/or value detected in a sample of a patient that is not immune to a particular pathogen. In one embodiment, such reference signal and/or reference value is a signal and/or value detected in a sample in which no pathogen-targeting compound is present. In one embodiment, a method of detecting a pathogen-neutralizing molecule comprises determining a presence of a pathogen-neutralizing molecule, preferably antibody, if a signal detected in step iv) is increased compared to a reference signal and/or reference value. In an alternative embodiment, a method of detecting a pathogen-neutralizing molecule comprises determining a presence of a pathogen-neutralizing molecule, preferably antibody, if a signal detected in step iv) is decreased compared to a reference signal and/or reference value. In one embodiment, said increase or decrease depends on whether said signal is directly or indirectly detected, i.e. depending on whether a signal of the released marker or a signal of the PTP-comprised marker is measured. In one embodiment, a kit comprises instructions for determining a presence of a pathogen-neutralizing molecule, preferably antibody, if a signal of a marker is increased compared to a reference signal and/or reference value. In an alternative embodiment, a kit comprises instructions for determining a presence of a pathogen-neutralizing molecule, preferably antibody, if a signal of a marker is decreased compared to a reference signal and/or reference value.
In one embodiment, a signal of the marker is reduced and/or quenched as long as said marker is comprised in the pathogen-targeting particle, preferably such signal is only detected when released from the pathogen-targeting particle e.g. released due to lysis of said pathogen-targeting particle; accordingly, if a pathogen-neutralizing molecule is present in said sample, said pathogen-targeting particle is not lysed, said marker is not released from said marker, and thus, the signal of the marker is still reduced and/or quenched; accordingly, if a pathogen-neutralizing molecule is absent in said sample, said pathogen-targeting particle is lysed, said marker is released, and thus the signal increases.
In an alternative embodiment, a signal of the marker is detectable and/or is significantly detectable when the marker is comprised in the pathogen-targeting particle, the pathogen-targeting particle preferably being immobilized, preferably said signal of the marker is detected when the marker is comprised in the pathogen-targeting particle; accordingly, if a pathogen-neutralizing molecule is present in a sample, said pathogen-targeting particle is not lysed, said marker is not released from said particle, and thus, the signal of the marker remains intact; accordingly, if a pathogen-neutralizing molecule is absent in said sample, said pathogen-targeting particle is lysed, said marker is released, and thus the signal decreases. In an alternative embodiment, a signal of the marker is detected in a supernatant when the marker is released from a pathogen-targeting particle; accordingly, if a pathogen-neutralizing molecule is present in a sample, said pathogen-targeting particle is not lysed, said marker is not released from said marker, and thus, the signal of the marker is low and/or absent in said supernatant, preferably absent; accordingly, if a pathogen-neutralizing molecule is absent in said sample, said pathogen-targeting particle is lysed, said marker is released, and thus the signal increases.

In one embodiment, depending on the assay format used, e.g. depending on whether the pathogen-targeting particle is immobilized or not, and/or depending on whether a marker is used that is quenched when comprised in the pathogen-targeting particle, the marker signal detected, if a pathogen-neutralizing molecule is present, is decreased or increased compared to a reference signal and/or reference value. A person skilled in the art in view of the present invention understands how to set up the assay for performing a method of the invention. In one embodiment, if the pathogen-targeting particle binds to the pathogen-mimicking particle, the lysis of the pathogen-targeting particle is triggered, for example via a complement activating agent, and the marker is released from the pathogen-targeting particle due to said lysis. In one embodiment, if a pathogen-neutralizing molecule is present, the pathogen-targeting particle does not bind to the pathogen-mimicking particle, therefore, the pathogen-targeting particle is not lysed and the marker is not released from said pathogen-targeting particle. In one embodiment, the complement activating agent is incorporated in the pathogen-mimicking particle, binds to the pathogen-mimicking particle, and/or binds to a PTP-PMP complex, and thereby is only contacted with said pathogen-targeting particle, if the pathogen-targeting particle binds to the pathogen-mimicking particle. The term "PTP-PMP complex", as used herein, relates to a pathogen-targeting particle and a pathogen-mimicking particle bound to each other.

In one embodiment, a method of the invention is a bioassay comprising a pathogen-targeting particle which is a liposome, wherein said pathogen targeting molecule is a receptor or an enzyme, e.g. ACE2 or TMPRSS2. In one embodiment, the method of the invention is a point-of-care (POC) neutralization assay for pathogens such as SARS-CoV-2. In one embodiment, an assay is, for example, a colorimetric, fluorescent, chemiluminescent, electrochemiluminescent, and/or electrochemical assay.

An aim of the invention is to provide a method for detecting pathogen-neutralizing molecules, e.g. a cell-free receptor binding virus neutralization test, preferably for high throughput applications and/or for on-site testing, such as rapid on-site testing using a POC device. In one embodiment, pathogen-targeting particles, e.g. long-term stable, marker-filled liposomes, are labeled with a pathogen-targeting molecule, e.g. recombinant ACE2, and such pathogen-targeting particles are added, preferably together with pathogen-mimicking particles such as SARS-CoV2-like particles (VLPs), to a sample such as patient serum. In one embodiment, neutralizing patient antibodies inhibit a binding of a pathogen-mimicking particle and a pathogen-targeting molecule, e.g. binding of a VLP and ACE2. In one embodiment, in case such inhibition of a binding between a pathogen-mimicking particle and a pathogen-targeting particle, e.g. VLP binding to ACE, does not take place due to an absence of a neutralizing antibody, a PMP-PTP complex, e.g. a liposome-VLP complex, is formed. In one embodiment, a pathogen-mimicking particle specific, e.g. VLP-specific, non-neutralizing antibody is added as a complement activating agent.

In one embodiment, the binding of a non-neutralizing antibody, VLP-specific non-neutralizing antibody, or a complement activating agent other than a non-neutralizing antibody, to the PMP-PTP complex, e.g. liposome-VLP complex, to the pathogen-targeting particle, and/or to the pathogen-mimicking particle triggers the complement system present in a sample, such as serum. In one embodiment, such activated complement system lyses pathogen-targeting particles, e.g. liposomes, thereby releasing marker entrapped in the pathogen-targeting particles, e.g. liposome. In one embodiment, the marker can be and/or is immediately detected after lysis.

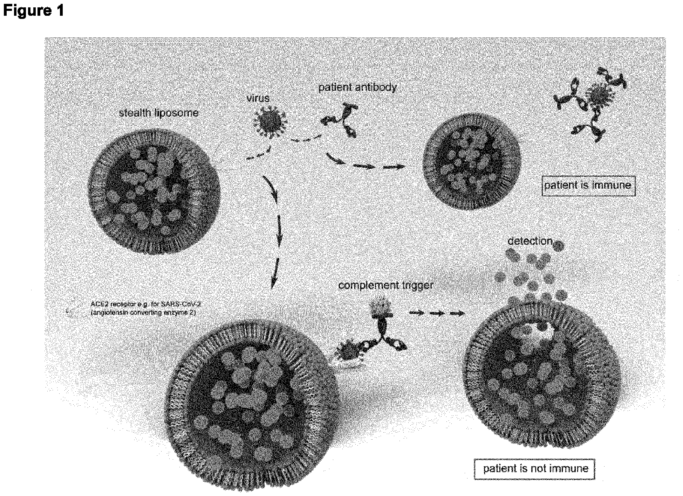

The present inventors provide a method, e.g. a receptor-based assay, based on a particle collection, particularly pathogen-targeting particles, e.g. using liposome technology, pathogen-mimicking particles, e.g. virus-like particles, and complement activating agents, e.g. antibodies capable of activating the complement system (Figure 1). Key innovative aspects are (i) the multi-functional assay format allowing for high-throughput testing and/or point-of-care testing (POCT), (ii) liposomes that can be loaded with various options of marker molecules, allowing for significant signal enhancement and various readout strategies, (iii) VLPs presenting pathogen proteins, such as SARS-CoV-2-S protein, and optionally additional proteins for universal recognition, which enable virus neutralization assays outside of biosafety level 2 facilities; (iv) non-neutralizing antibodies, e.g. pathogen specific antibodies, such as SARS-CoV specific antibodies, or universal antibodies tagged with complement activating moieties, enabling a rapid and homogeneous assay format; (v) a VLP that co-presents a complement activating moiety increases assay simplicity, reduces assay time and costs.

Various pathogens, such as SARS-CoV-2, are known to infect cells by initially binding to a cell receptor, such as the cellular ACE2 receptor using proteins, such as the S protein of SARS-CoV-2. In one embodiment, if a subject has developed antibodies against a pathogen, e.g. SARS-CoV-2 specific antibodies, such antibodies, specifically neutralizing antibodies, bind to the pathogen, e.g. to S protein displayed on the surface of a virus particle, and hinder the pathogens from infecting the subject's cells. Mechanisms of neutralizing antibodies are, for example, the inhibition of receptor binding, and/or blocking of penetration or uncoating of the virus particle.

In one embodiment, pathogen-targeting particles such as liposomes are tagged with a pathogen-specific protein, such as the ACE2 receptor protein. If antibodies present in a sample of a patient are able to prevent the pathogen e.g. virus from binding to its receptor, the patient has developed neutralizing antibodies in response to an earlier infection with said pathogen, e.g. an earlier SARS-CoV-2 infection. If a pathogen and/or PMP binds to a PTP-bound protein, such as a liposome-bound ACE2 receptor, liposome lysis is triggered, which can easily be detected, e.g. using a method of the present invention (Figure 1). Such lysis indicates that the patient has had no exposure to said pathogen, e.g. said SARS-CoV-2, before, and that said patient is not immune. The method of the invention allows for rapid and clear results indicating whether a patient has developed neutralizing antibodies. Furthermore, a method of the invention, such as an in vitro method of the invention, allows to determine whether a patient is immune to a pathogen or not.

The method of the invention can be an assay provided in different formats, particularly there are three different assay strategies, e.g. liposome assay strategies, which can serve as high throughput assay in clinical testing labs and which allow easy screening, e.g. of the entire population, via POCTs in doctors' offices and pharmacies, preferably assay formats such as fluorescent microtiter plate assays (A), electrochemical assays (B), and/or LFA (C). Fluorescent microtiter plate assays (A) are designed for high throughput bench-top assays for clinical labs. Electrochemical sensors (B) are a second generation assay. Their design is an on-site version of the fluorescent assay. Finally, a visionary LFA (C) has an immense benefit gained, as LFAs do not require any equipment and are ultimately simple to use.

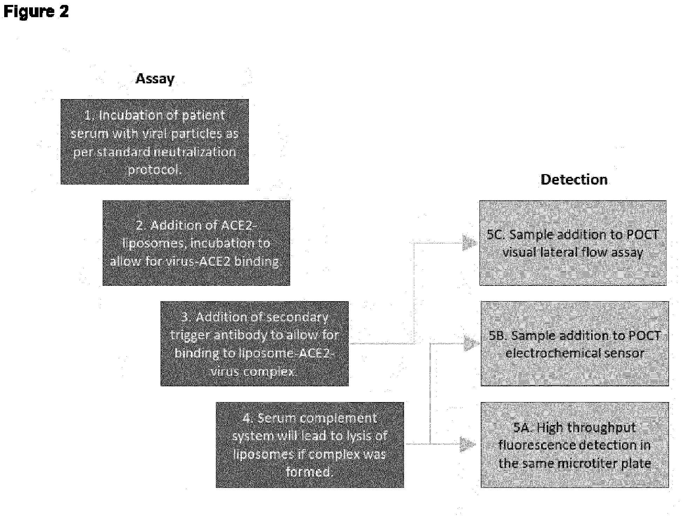

In one embodiment, liposomes are stabilized, e.g. to be stable in serum, via pegylation and/or liposome size control. In one embodiment, by integration of a complement activating molecule, liposome lysis can be specifically triggered. In one embodiment, complement induces specific lysis of pegylated liposomes, if triggered through antibody binding. Thus, a method of the invention, for example a liposome bioassay, is straightforward (Figure 2) and only requires a PTP, PMP, and optionally a complement activating agent, for example a liposome, a VLP, and an antibody optionally tagged with an appropriate complement activator (trigger antibody), and a sample to be tested, such as a serum sample. In one embodiment, the terms "assay" and "method of detecting" are used interchangeably.

As also outlined in Figure 2, a method of the invention may comprise one or more of the following steps:
1. Incubating a patient's sample, such as a serum sample, with a PMP, e.g. a non-infectious virus and/or virus-like particle (VLP). In one embodiment, a serum sample is an inactivated and/or diluted sample. In case antibodies are present in said sample which can bind to and neutralize the non-infectious virus, such antibodies neutralize the virus.
2. Adding liposomes and optionally subsequent incubation. In case no neutralizing antibodies are present in the sample, free PMPs bind to pathogen-targeting molecules, e.g. an ACE2 receptor.
3. Adding compliment activating agent e.g. an anti-pathogen antibody optionally tagged with a complement-activating molecule. The compliment activating agent e.g. antibody binds to PMP bound to the pathogen-targeting molecule, e.g. the ACE2 receptor.
4. If the antibody binds to PMP bound to the pathogen-targeting molecule, the complement system of the patient serum is triggered, and the liposomes present in a liposome-[pathogen-targeting molecule]-PMP-antibody complex, e.g. a liposome-ACE2-S-VLP-antibody complex, are lysed.
5. Markers are released from the lysed liposomes and can be detected, e.g. using fluorescence, electrochemistry, visual detection, and/or detection using a cell phone.

In one embodiment, in the case of a high throughput microtiter plate fluorescence assay (A), all steps are done within one microtiter plate. In one embodiment, reagents are added consecutively to the plate with intermediate incubation steps, and optionally intermediate washing steps. In one embodiment, no washing steps are needed. In one embodiment, for the electrochemical POCT (B), the same process is followed as described for (A) above, wherein incubation can be done in a vial. In one embodiment, for detection, the sample is added to a sensor device, e.g. a POC device. In one embodiment, for the POCT lateral-flow assay (C), incubation in a container, such as a vial or flask, is followed by addition to the LFA at an earlier part of the incubation sequence than for (A) or (B).

In one embodiment, two general types of liposomes are (1) stealth liposomes that cannot be lysed by the complement system, unless PMP, e.g. virus particles, bind to the pathogen-targeting molecule, e.g. ACE2, and the complement activating agent, e.g. antibody, binds; and (2) non-stealth liposomes that are lysed by the complement system and function as controls in the final product. Non-stealth liposomes are optimized to remain stable upon storage and lyse upon contact with the complement system in serum. In a preferred embodiment, liposomes are lysed quickly by the complement system and with a high yield upon activation via a complement activating agent. A complement activator can be additionally incorporated in the non-stealth liposomes to further enhance lysis via the complement system. In one embodiment, liposome lysis is monitored using fluorescence detection in a microtiter plate format and/or using chemiluminescence, such as mCOOH-luminol, and/or using a LFA. In one embodiment, liposome lysis can be detected using several techniques, such as fluorescence, chemiluminescence, and LFA, e.g. by incorporating one or several markers. Furthermore, stealth liposomes, e.g. pegylated and/or small stealth liposomes, are optimized to guarantee tight size distributions.

In one embodiment, an incubation step is between 0 and 60 minutes. In one embodiment, heat-inactivated serum is used as negative control to ensure that lysis is triggered by the complement system. The synthesis of liposomes is typically performed using the common reverse phase evaporation method. For example, dipalmitoyl fatty acid components and choline (20%), glycerol (20%), and choline modified with polyethylene glycol (10%), are mixed with 40% cholesterol. A high cholesterol content, such as 40% cholesterol, ensures long-term stability, and the glycerol headgroup ensures negative charge and hence colloidal stability. In one embodiment, fluorescent dye SRB is dissolved in HEPES buffer containing 150 mM NaCl. In one embodiment, purification of the liposomes is accomplished via gel filtration and dialysis against a HEPES+NaCl buffer adjusted to appropriate osmolality with sucrose. In one embodiment, liposomes are characterized by standard methods including ICP-OES to determine lipid concentration, DLS to determine their hydrodynamic diameter, and/or fluorescence to determine the fluorescent dye entrapment yield which indicates the liposomes' signaling power. In one embodiment, small variations between synthesized lots, if present, can be compensated for, since the concentration and signaling power of the liposomes can be quantified and can be adjusted accordingly prior to the initial use.

In one embodiment, a method of detecting comprises contacting and/or incubating a sample with a composition of the invention and/or at least two components of a particle collection of the invention, wherein said components can be added to the sample simultaneously or consecutively.

### Expression of a pathogen-targeting molecule, such as SARS-CoV-2 receptor Angiotensin converting enzyme (ACE) 2

In one embodiment, the pathogen-targeting molecule, e.g. ACE2, comprises a transmembrane domain and a domain presented on the surface of a membrane, e.g. a cell-bound ACE2 contains a transmembrane domain and a soluble domain. Accordingly, the domain presented on the surface can be bound by the PTP, PMP and/or pathogen, such as a virus. In one embodiment, presentation of such soluble domain is sufficient to provide binding of a pathogen-targeting molecule, e.g. ACE2, to a pathogen and/or PMP, e.g. via a spike protein. In one embodiment, a codon-optimized plasmid can be used to provide a pathogen-targeting molecule, e.g. a pathogen-targeting molecule which is a soluble domain such as the soluble domain of ACE2. In one embodiment, such pathogen-targeting molecule and/or a biomarker can be expressed in any suitable cell line, such as in the HEK293 or Expi293 cell lines. In one embodiment, a recombinant pathogen-targeting molecule, such as recombinant ACE2, is purified using His-tag-specific affinity chromatography followed by gel filtration. In one embodiment, protein quality control is performed using SDS-PAGE, protein staining, and/or immunoblotting. In one embodiment, an existing His-tag can be exchanged for the sorptase cloning strategy. In one embodiment, such strategies allow for gentle and efficient coupling of pathogen-targeting molecules, such as ACE2, to liposomes. In one embodiment, an Avitag is used for enzymatic and gentle biotinylation of a recombinant pathogen-targeting molecule, such as ACE2.

### Synthesis of biotinylated PEG-coated small liposomes tagged with a pathogen-targeting molecule, such as an ACE2 receptor.

Three exemplary strategies of preparing liposomes are:
(a) Preparation of pegylated liposomes to be covalently tagged with streptavidin following standard protocols. For example, streptavidin groups can be coupled to COOH-groups on a pegylated liposome surface via standard NHS/EDC chemistry. Subsequently, a pathogen-targeting molecule such as ACE2 is biotinylated using biotin-NHS, and is incubated with liposomes for 1-60 min, e.g. 15 minutes. This process is very gentle on the unmodified pathogen-targeting molecule, such as an unmodified ACE2 protein. In one embodiment, a recombinant ACE2 is prepared with an Avitag for subsequently achieving biotinylation enzymatically.
(b) Preparation of pegylated liposomes to be covalently tagged with a pathogen-targeting molecule, e.g. ACE2, through standard NHS-EDC chemistry.
(c) Pegylated liposomes are covalently modified with pentaglycine. This serves as first coupling substrate for the enzyme sortase. A pathogen-targeting molecule such as ACE2 is modified with the LPXTG peptide as second sortase substrate and thus can be enzymatically covalently linked to the liposome surface. In one embodiment, the presence of a pathogen-targeting molecule such as ACE2 on the liposome surface is verified using an antibody test developed for the detection of soluble pathogen-targeting molecule, e.g. ACE2, and/or using a commercially available ELISA kit.

### Secondary antibody labeling system

For the secondary antibody labeling system four characteristics have to be full filled:
a) the antibody needs to bind to a pathogen and/or PMP, e.g. SARS-CoV-2 (e.g. non-infectious virus-like particles as described herein),
b) it needs to locally activate the complement system, when it is bound to the virus,
c) it does not interfere with virus binding to ACE2; and
d) it needs to be available in a reasonable amount.

The possibility of antibodies to function as a complement activating agent and to lead to liposome lysis has already been proven in drug delivery studies.

In one embodiment, a complement activating agent such as an anti-pathogen antibody, e.g. an anti-SARS-CoV-2 antibody binding to the S protein, is expressed in HEK cells with the ability to easily and efficiently produce high amounts such as 100 mg in one setting. Such production can easily be upscaled. In one embodiment, commercially and/or science-network available pathogen-specific antibodies, such as anti-coronavirus monoclonal/recombinant antibodies, can be tested with regard to their complement activation potential. For example, if the pathogen to be tested is a coronavirus, anti-coronavirus antibodies are offered by, e.g. Sigma-Aldrich (clone 541-8F), AntibodiesOnline (ABIN2000065) or several research teams. In one embodiment, mouse hybridoma cell lines producing monoclonal antibodies specific for artificial peptide-conjugates and/or PMP are generated. In one embodiment, an integration of these peptides into a PMP, for example the capsid of a SARS-CoV-2-like particle, serves as a generic complement activating agent, independent from the PTP-PMP interaction, for example, independent from the ACE2-virus interaction. In one embodiment, generic complement-based liposome-PMP(e.g. virus)-complex lysis further broadens the application of this cell-free virus-neutralization assay independent from virus specific antibodies. In one embodiment, a complement activating agent is a molecule incorporated into the pathogen-mimicking particle and/or is a molecule binding to a molecule e.g. peptide of the pathogen-mimicking particle, and is independent of the particular pathogen mimicked and/or targeted. Thus, the particle collection of the invention is a universal assay toolkit which can be adapted to the respective pathogen. In one embodiment, the invention relates to a toolkit which can be adapted for various pathogens. In one embodiment, if a single complement activating agent such as a monoclonal antibody does not sufficiently trigger the complement system for liposome lysis autonomously, it may be subjected to in vitro coupling strategies to enhance the complement activation potential, e.g. (i) recombinant expression of antibody multimers, (ii) cloning of single chain variable fragments of the identified mAb-binding regions into a complement activating constant antibody region (e.g. IgM, pFUSE-CHIg-hM), and/or (iii) conjugate complement activating moiety such as carbohydrates or LPS-containing labels to the Fc-part of the antibody. Antibodies and modified antibody fragments can be characterized in classical immunological assays (ELISA, Western Blot) and can be tested in functional complement assays (complement deposition assay, anaphylatoxins detection). In one embodiment, the complement activation potential of a complement activating agent, e.g. an anti-SARS-CoV-2 non-neutralizing antibody, is tested by covalently binding the SARS-CoV-2 S protein, or artificial peptides to stealth, pegylated liposomes. Optimized liposomes can be coupled to the proteins or peptides, e.g. via EDC-NHS chemistry.

### Development of PMP, such as non-infectious virus-like particles (VLP)

Lentiviral non-infectious VLPs. HEK 293 cells, once transfected with a lentiviral, RNA- and codon-optimized Gag-gene, readily express a Gag precursor which is guided, amongst others, by the ESCRT complex to the inner leaflet of the plasma membrane and released as non-infectious virus-like particle into the cell culture supernatant in a budding-like process. Such VLPs are, similar to coronaviruses, enveloped by a membrane of cellular origin, incorporate some cellular membrane proteins such as CD46, mediating complement resistance, and resemble immature lentiviral particles in size (100-150 nm diameter) and shape. If co-expressed in a mammalian cell together with e.g. viral envelope protein, such as the CoV S protein, the envelope protein is incorporated into the particle. The present inventors herein provide purified Gag VLPs pseudotyped with the complete, non-engineered CoV2 S protein as reference VLPs. In order to increase the number of native S protein trimers displayed on such VLPs, the ER retention signal is either removed from the S protein cytoplasmic domain or, alternatively, the autologous transmembrane and cytoplasmic domain are substituted by a heterologous, VSV derived transmembrane domain. The best-in-class S protein variant is rigidified by introducing stabilizing mutations essentially as described previously in order to "freeze" conformational and potentially neutralizing epitopes. In one embodiment, a pathogen-mimicking particle is a virus-like particle, such as a pseudotyped virus-like particle. In one embodiment, a virus-like particle is a particle comprising one or more, several, or all noninfectious components of a virus.

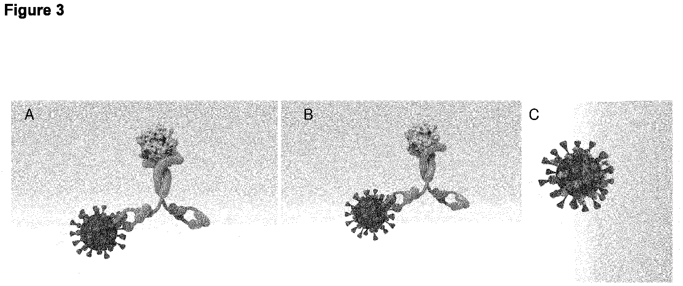

VLP with multi-functionalities: To reduce the complexity of the assay format and to further tune such particles with regard to the lysis of pathogen-targeting particles, e.g. ACE2 receptor equipped liposomes, a complement compound, such as a hexameric Fc fragment, can be co-displayed together with the above described S protein derivatives, thus avoiding the need for adding the secondary complement trigger antibody. This can be achieved by triple transfection of HEK293 cells with (i) RNA and codon optimized Gag construct, (ii) one of the above S-protein derivatives together with (iii) a mammalian expression construct encoding a generic epitope or peptide (see below) in a form enabling display on the VLP surface. Also, as a further strategy, VLPs will be displayed with the above described S protein derivatives and in addition one generic protein/peptide, e.g. FHR-1, ARMS2, FoxP or BBS6 peptides. In this case, only the receptor and the virus-specific protein on the VLP have to be generated and plugged into the liposome assay. The three VLP approaches are shown in Figure 3. In one embodiment, a complement activating agent is incorporated into the pathogen-mimicking particle, e.g. VLP, and/or a generic protein/peptide, e.g. FHR-1, ARMS2, FoxP, and/or BBS6, is incorporated into the pathogen-mimicking particle, wherein such generic protein/peptide can be bound by a complement trigger such as a universal antibody and/or a non-neutralizing antibody.

### Nanoparticles as virus surrogates

Inorganic nanoparticles. The present inventors previously demonstrated that Silica based nanoparticles (SiNP) can be readily equipped with various biomarkers, e.g. model antigens including complex viral (HIV) envelope proteins via chemical coupling. Controllable variables include particle size as well as orientation, spacing and density of SiNP displayed envelope proteins. For example, the present inventors link either the stabilized version of the complete external domain of the S protein or the S protein receptor binding domain to the nanoparticle. Linkage of a biomarker to nanoparticle can be efficiently achieved by using NHS/EDC chemistry or via a sortase tag or His tags. In order to tailor such SiNPs to the desired assay format and to further simplify the system, the inventors further couple a complement activating agent, e.g. complement components such as hexameric Fc fragments, to such nanoparticles, e.g. SiNPs, together with a biomarker, for example one of the 2 S protein derivatives, essentially as described for the VLPs above. In one embodiment, such inorganic based nanoparticles may differ in size and shape from a pathogen, for example natural CoV2, or non-infectious VLPs. In one embodiment, such nanoparticles have similar neutralization characteristics as a pathogen or a VLP. In one embodiment, such nanoparticles are very practical in terms of usability (stability, shipment, cost of production etc.).

Self-assembling protein nanoparticles. In one embodiment, self-assembling nanoparticles allow the multimerization of e.g. viral envelope proteins with the goal to benefit from avidity gains. Such particles have the advantage of carrying a fixed number and geometry of proteins on a particle surface, e.g. the CoV S / S-RBD in a fixed number and geometry on the surface, thus allowing for highly reproducible preparations. Importantly, this facilitates binding of antibodies via avidity gains, thus potentially increasing the sensitivity. The most commonly used scaffolding moieties are derived from ferritin and lumazine synthase, which - upon transfection with the respective expression plasmids - self-assemble into 12.2 and 14.8 nm particles which are readily released then from transfected cells. Importantly, these properties are maintained when parts of viral envelope proteins such as e.g. a core domain of the HIV external glycoprotein gp120 are properly fused to these scaffolds. Various nanoparticles and/or pathogen-mimicking particles are possible, e.g. (i) presenting the CoV2 S protein RBD alone or (ii) together with the complement component hexameric Fc fragment. The latter can be achieved by co-expressing the scaffold-RBD together with the scaffold-complement-component, thereby yielding chimeric protein nanoparticles which trigger direct complement mediated lysis in absence of neutralizing antibodies. In one embodiment, a polymeric nanoparticle and/or an organic nanoparticle is a protein nanoparticle.

### Liposome-based fluorescent high-throughput bioassay

In one embodiment, a method of the invention comprises a fluorescent marker and/or detecting a fluorescent signal. For example, a method involving a fluorescent microtiter plate assay is carried out as indicated in Figure 2. Specifically, patient serum (diluted or not diluted) is mixed with viral particles as per standard neutralization procedures. Subsequently, liposomes are added to enable non-neutralized viruses to bind to the ACE2 receptor. Finally, a complement activating agent, e.g. a secondary trigger-labeled antibody (trigger antibody), is added, and the fluorescence of the released fluorescence marker following PTP lysis, e.g. liposome lysis is measured. Lysis of PTPs e.g. liposomes indicates that PMP, such as virus-like particles, have bound to the pathogen-targeting molecule, e.g. the ACE2 receptor, and thus indicates that a patient lacks neutralizing antibodies against the pathogen, e.g. SARS-CoV-2. In one embodiment, the incubation times of PTP e.g. liposomes with a sample e.g. serum, the incubation time of complement activating agent e.g. secondary antibody with the PTP-PMP complex, the amount of PTP, and the concentration of complement activating agent e.g. trigger antibody can be optimized according to methods known to a person skilled in the art. In one embodiment, additional reagents can be added to further enhance assay performance and particle e.g. liposome stability (such as sugars, salts, polymers).

Positive and negative controls: A positive control can accomplished by testing patient serum without the addition of a complement activating agent, a trigger-labeled antibody, and/or without the addition of a PMP. Hence, pegylated liposomes should remain intact and no signal should be obtained. Additionally or alternatively, a PTP without a pathogen-targeting molecule, e.g. a pegylated liposome without ACE2 receptor, can be used as positive control. Two negative controls may be performed: (a) detergent (e.g. Triton X-100) can be added to the sample e.g. patient serum, and ensures that a high signal is obtained, as PTP e.g. liposomes are lysed. This ensures the functionality of PTPs such as liposomes. (b) non-pegylated liposomes can be added to the sample e.g. patient's serum. If the patient's complement system is active, these are lysed. This provides a control for the complement activating agent. In one embodiment, a pathogen-targeting particle is pegylated. In one embodiment, one or more controls can be used, for example 1) a complement activating agent binding to PTPs independent of PMP presence (negative control, as it leads to liposome lysis), e.g. complement activating agents that bind to liposomes independent of VLP presence, 2) inactive serum (positive control, as it does not lead to liposome lysis), 3) incubation of PTP, preferably liposomes, in detergent (negative control; may provide maximum possible signal depending on the assay format), 4) PTP without pathogen-targeting molecules (positive control, as no PTP is lysed).

### Electrochemical point-of-care sensor

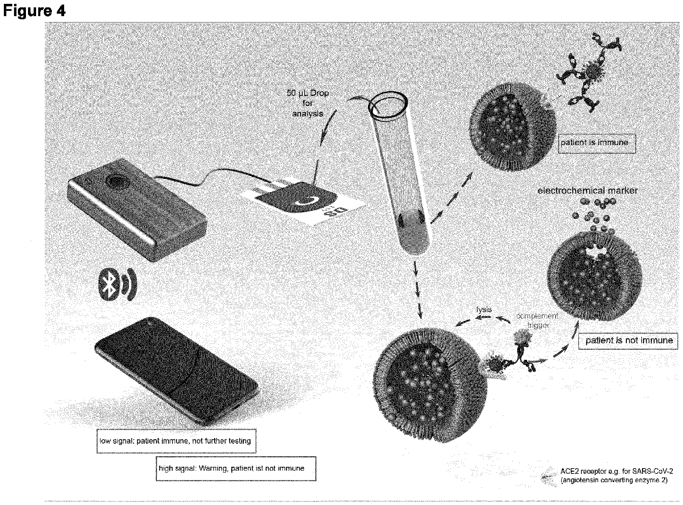

A method of the invention, for example involving a fluorescent liposome assay, can be used for a POCT sensor concept. Accordingly, a method of the invention can be a POC method of detecting a pathogen-neutralizing molecule. A pathogen-targeting molecule can comprise any kind of marker, such as a fluorescent dye or an electrochemical marker, i.e. 150 mM potassium hexaferricyanide. An electrochemical marker enables electrochemical detection which provides the same level of detection limit as that obtained with fluorescent liposomes, and can be carried out on single-use, inexpensive electrodes and run by a small portable potentiostat device. Data can be recorded either on the potentiostat or on a cell phone, e.g. via App and Bluetooth connection. First, PTP e.g. liposomes are synthesized. The assay protocol is adapted to the volume needed for a marker and/or detection system, e.g. electrochemical detection (e.g. 50 - 100 µL per analysis). Such electrochemical assay (Figure 4) follows the same strategy as used for a fluorescent assay. In one embodiment, no microtiter plate is needed. In one embodiment, a sample is incubated, preferably with a particle collection, e.g. in separate vials, and then directly added to the electrode. Again, a high signal indicates that the patient has no neutralizing antibodies and the patient is thus presumably not immune. A low signal or absence of a signal indicates that the patient has neutralizing antibodies against the pathogen, e.g. SARS-CoV-2. Positive and negative controls, as described above, can be used. The advantage of an electrochemical POCT approach is that it can be performed outside of a central testing lab, such as in a doctor's office, and no expensive equipment is needed. In one embodiment, such assay is performed as single test directly on-site. Furthermore, if a range of VLP is used, an antibody titer can be determined in a quantitative fashion just like in a high-throughput microtiter plate assay.

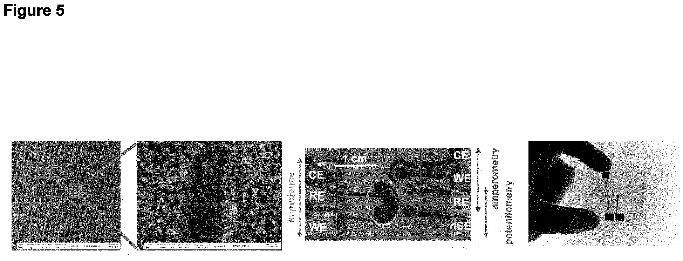

In one embodiment, commercial screen-printed electrodes, e.g. from DropSense, are used and/or self-made electrodes are used prepared using laser scribing. Laser scribing generates highly sensitive nanostructured laser-induced graphene (LIG) electrodes (Figure 5). Such electrodes may outperform commercially available screen-printed electrodes commonly used in the current diagnostic market (such as those used for most glucose tests). Furthermore, they can be mass produced in a roll-to-roll method and be integrated into microchips (much in contrast to screen-printed electrodes). In one embodiment, electrodes may be coated to avoid any non-specific signals through serum components, such as using Nafion or chitosan membranes, or blocking via bovine serum albumin (BSA), polyvinyl pyrrolidone (PVP), which are standard blocking strategies for electrochemical analyses in blood samples.

Furthermore, the electrochemical POCT can be integrated into a microfluidic chip, e.g. in a capillary driven microfluidic device. In one embodiment, a method, kit, and POC device of the invention are advantageous in that an entire incubation of a sample such as patient serum, viral particles, liposomes and trigger antibody can be performed within one POCT device. In one embodiment, the terms "POC" and "POCT" are used interchangeably for point-of-care (testing).

### Point-of-care lateral flow assay

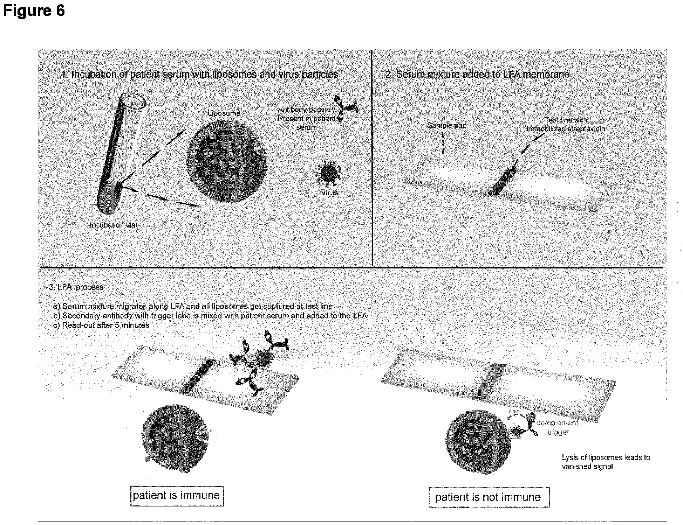

In one embodiment, a PTP comprises a marker which is sulforhodamine B (SRB). In one embodiment, SRB entrapping PTP e.g. liposomes can be used for colorimetric detection, optionally further comprising a biotin tag on the PTP surface. Such marker and/or colorimetric detection is highly advantageous in that respective data recording can be done with the bare eye (no device needed) or with a suitable detection system, e.g. a cell phone camera. The assay protocol can be slightly changed from the fluorescent and electrochemical approaches, if necessary, since the complement triggered lysis may take place on a lateral flow assay (LFA) membrane (Figure 6). In one embodiment, a method of detecting relates to an assay, and vice versa. In one embodiment, a method of detecting is and/or involves a point-of-care method, an electrochemical assay, a lateral flow assay, and/or a high-throughput method, e.g. using fluorescent microtiter plate assays. In one embodiment, a method of detecting of the invention is configured to detect a signal of a marker using any of a point-of-care assay, an electrochemical assay, a lateral flow assay, and/or a high-throughput method, e.g. using fluorescent microtiter plate assays. In one embodiment, when a method of detecting of the invention is configured to comprise a high throughput detection of said signal of said marker, said method is configured to be performed with or without, preferably without, an intermediate washing step.

In one embodiment, upon incubation of a sample such as patient serum, with any of PTP and PMP, e.g. with viral particles and liposomes, the mixture is added to a LFA membrane. In one embodiment, PTPs such as liposomes are immobilized on a surface, for example a test line, through any suitable immobilization means, e.g. biotin-streptavidin binding. Next, a complement activating agent, e.g. a secondary trigger antibody, is added to the LFA membrane. If PMP and/or a pathogen is present on the PTP e.g. liposomes, the complement activating agent binds thereto, and the complement triggers a lysis of the PTP liposomes. The released marker e.g. SRB dye migrates up the strip and the signal of the test line thus vanishes. Hence, in such assay, the vanishing of a signal and/or absence of a signal in the test line indicates that patients lack neutralizing antibodies and may therefore be assumed as being not immune. The presence of the signal indicates that patients have effective neutralizing antibodies against the pathogen, e.g. SARS-CoV-2, as intact PTP e.g. liposomes are bound to the test line e.g. streptavidin-line. Such intact PTP still comprise the marker and thus provide a signal, since, due to the presence of pathogen-neutralizing molecules, PTP-PMP complexes have not been formed and said PTP thus have not been lysed. In one embodiment, the marker signal which is detected is a signal of marker comprised by said PTP. In an alternative embodiment, the marker signal which is detected is a signal of a marker released from said PTP. Accordingly, the lysis of a PTP, and thus the absence of a pathogen-neutralizing molecule, can be detected directly or indirectly, depending on whether a signal of the released marker or a signal of the PTP-comprised marker is measured.

In one embodiment, a POC method preferably comprises using an appropriate positive and negative control as set forth above. In one embodiment, the positive control contains complement activating agent. In one embodiment, if the PTP is a liposome, the negative control(s) is/are non-pegylated liposomes and/or normal pegylated liposomes plus a detergent (e.g. Triton X-100). In one embodiment, the LFA assay is a 2-step LFA in which a PTP is immobilized on a surface, a first step is incubating the sample with a PMP and/or pathogen and adding such sample-PMP/pathogen mixture to the LFA, and a second step is adding a complement activating agent to the LFA.

Optionally, further internal assay controls can be added. In one embodiment, a pH indicator is added to the sample and/or sample pad. In one embodiment, a pH indicator such as phenolphthalein turns to a different color upon entering a waste pad. In one embodiment, negative control PTPs, for example negative control liposomes, may be added to the sample. In one embodiment, negative control PTPs are not tagged with a pathogen-targeting molecule, e.g. the ACE2 receptor, and/or not tagged with biotin, and instead comprise a label such as a fluorescein or digoxygenin label. In one embodiment, in a control line, anti-fluorescein or anti-digoxygenin antibodies are immobilized. The successful completion of the assay is indicated, if the control line presents a signal. In one embodiment, for detecting a signal of a marker, images are taken using a cell phone camera. Numerous systems are already available and can be adapted to this assay.

In one embodiment, a method of the invention is an assay which is an investigative (analytic) procedure. In one embodiment, the method of the invention is a high throughput assay, an electrochemical assay, a lateral flow assay, and/or a point-of-care assay. For example, a method of the invention may relate to and/or involve a liposome receptor-based assay. In one embodiment, a method of the invention is an assay based on and/or using a particle collection and/or composition of the invention. In the following, characteristics of three exemplary liposome receptor-based assays are described:

### High throughput screening:

| **Measure** | **Target** | **Liposome assay** |
|---|---|---|
| Biosafety measure | performed outside of BSL2, and hence possible in standard analytical laboratories | Yes, e.g. through surrogate VLP system |
| High throughput | Similar to ELISA | Performance can be done in microtiter plates using standard fluorescence plate readers. For example, format in 96 well plates. Expansion to 384 well plates is also envisioned. |
| Assay time | Similar or less than ELISA | 1 - 2 hours |
| Storage | Storage at 4 and -20 °C for 1 year | Long-term storage of liposomes at 4 °C for >1 year. Storage of complement system, proteins and VLPs at - 20 °C is known. |

### Electrochemical POCT:

| **Measure** | **Target** | **Liposome assay** |
|---|---|---|
| Biosafety measure | performed outside of BSL2, and hence possible in on-site, e.g. in doctors' offices | Yes, e.g. through surrogate VLP system |
| POCT | Similar to glucose sensor | Patient serum will be mixed with assay reagents in a container, e.g. vial. It will then be added to the electrode for detection. Readout is done e.g. via cell phone app or potentiostat reader. |
| Assay time | Results obtained same day | 1 - 2 hours |
| Storage | Storage at 4 and -20 °C for 1 year | Long-term storage of liposomes at 4 °C for >1 year is known. Storage of complement system, proteins and VLPs at -20 °C is known. |

### Lateral-flow assay POCT:

| **Measure** | **Target** | **Liposome assay** |
|---|---|---|
| Biosafety measure | performed outside of BSL2, and hence possible in doctors' offices, health clinic, pharmacies | Yes, e.g. through surrogate VLP system |
| POCT, e.g. in doctors' offices, health clinics, pharmacies | Similar to home pregnancy test | Patient serum is mixed with assay reagents in container, e.g. vial. It is then added to the LFA for detection. Readout is done via cell phone app. |
| Assay time Storage | Results obtained <1 Storage at 4 and -20 °C for 1 year | 30 - 60 minutes Long-term storage of liposomes at 4 °C for >1 year is known. Storage of complement system, proteins and VLPs at -20 °C is known. |

As used herein, the term "comprising" is to be construed as encompassing both "including" and "consisting of', both meanings being specifically intended, and hence individually disclosed embodiments in accordance with the present invention. Where used herein, "and/or" is to be taken as specific disclosure of each of the two specified features or components with or without the other.

### BRIEF DESCRIPTION OF THE FIGURES

The present invention is now further described by reference to the following figures.

All methods mentioned in the figure descriptions below were carried out as described in detail in the examples.
**Figure** 1 shows an assay principle of a method of the invention, for example a liposome-based test, to detect a pathogen-neutralizing molecule, e.g. to determine patient immunity against a pathogen such as SARS-CoV-2. First, PMP are added to patient serum. Patient antibodies, if present, bind to the PMP and/or virus and 'neutralize' it. Then, PTP e.g. liposomes are added as shown here. Liposomes are marker filled nanovesicles and are tagged with a pathogen-targeting molecule e.g. ACE2. If the virus is neutralized by patient antibodies, it cannot bind to the respective receptor. If it is not neutralized, it binds to the ACE2-liposome complex. Upon subsequent binding of a complement activating agent, e.g. a complement triggering antibody, such liposomes are lysed by serum complement components thereby indicating the presence of a non-neutralized virus. In this context, the term 'immune' refers to the fact that patients have pathogen-neutralizing molecules, e.g. neutralizing anti-pathogen antibodies, such as anti-SARS-CoV-2 antibodies, capable of blocking the binding between a virus and a receptor.
**Figure 2** shows exemplary workflows of a method of the invention, particularly three different exemplary detection strategies that can be used in a method of the invention.
**Figure 3** shows exemplary PMPs e.g. VLP constructs: (A) VLP with co-expressed S-Protein, which is recognized by anti-SARS-CoV-2 antibodies labeled with complement trigger; (B) VLP co-expressed with S Protein and generic protein, which will be recognized by its appropriate antibody labeled with complement trigger; (C) VLP with co-expressed S Protein and complement trigger, no additional antibody is needed in this system.
**Figure 4** shows an exemplary assay principle of a method of the invention, e.g. of an electrochemical POCT liposome assay. Patient serum, VLPs and liposomes are incubated in a vial (as in Fig. 1). Lysis of liposomes, and hence binding of the virus to its ACE2, is determined by adding a drop of the solution to a single-use electrode. A detecting device, such as a potentiostat, transfers the signal via bluetooth to a cell phone. An App can then report the findings to the local doctor and health authorities. Note: 'immune' refers to the fact that the patient has neutralizing antibodies available.
**Figure 5** shows laser-induced graphene electrodes. Left shows the nanostructured surface by SEM imaging with scale bars of 10 µm and 1 µm, middle shows a multi-analyte sensor enabling all relevant electrochemical techniques; right shows LIG electrodes integrated into microfluidic channels.
**Figure** 6 shows a principle of the lateral-flow liposome receptor assay. The term 'immune' refers to the fact that patients have neutralizing anti-SARS-VoV2 antibodies which are able to effectively inhibit virus binding to ACE2 and hence infection by the virus.

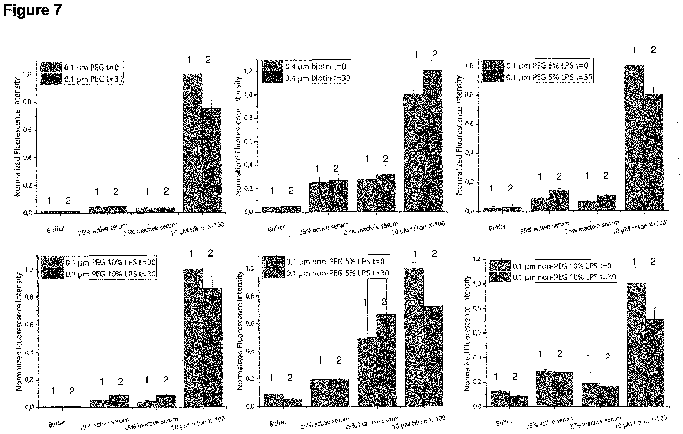

**Figure 7** shows a comparison of different liposome systems. Normalized fluorescence intensities of different liposome systems diluted in complement buffer to ∼75 µM total lipid concentration, with addition of 25% active and inactive serum, respectively, at ∼75 µM total lipid concentration and with addition of 10 µM triton X-100 at ∼75 µM total lipid concentration. Red bars represent measurements after the pipetting process and blue bars represent measurements after 30 min incubation. Intensities are normalized to triton X-100 t=0, n=2 or 3 due to low sample amount.

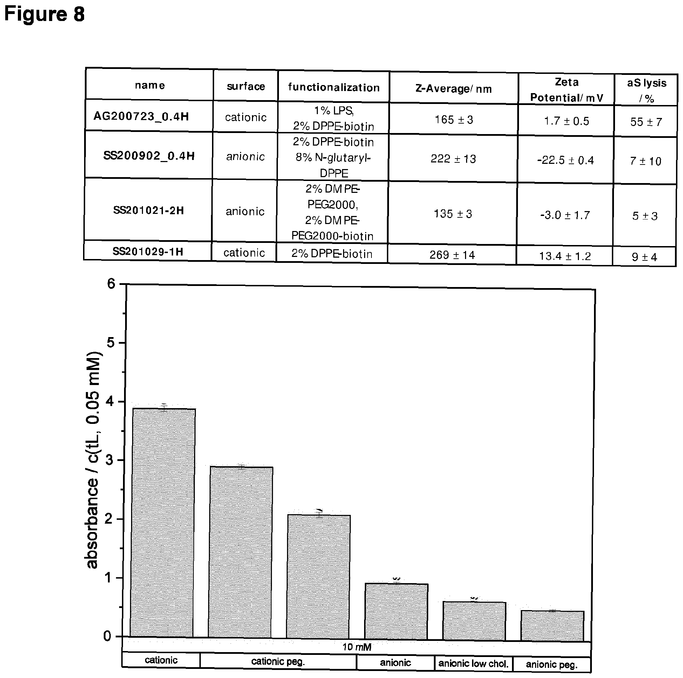

**Figure** 8 shows a characterization of exemplary pathogen-targeting particles, e.g. anionic or cationic liposomes, optionally PEG-modified, as described in Example 3.

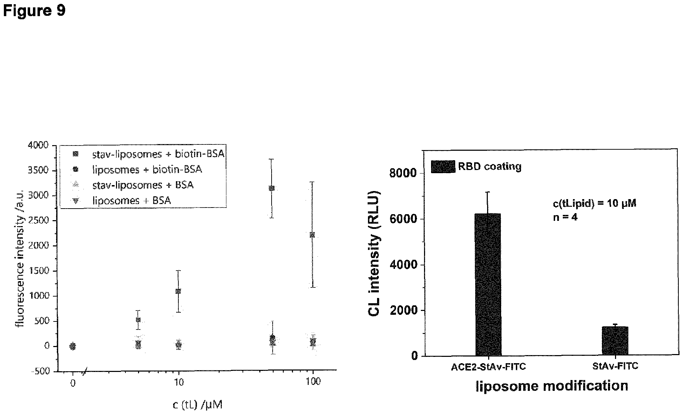

**Figure 9** shows protein coupling to pathogen-targeting particles, e.g. liposomes, as described in Example 3. Streptavidin(stav)-liposomes provided a dose response signal when bound to biotinylated BSA. Biotinylated ACE2 was successfully bound to streptavidin-liposomes.

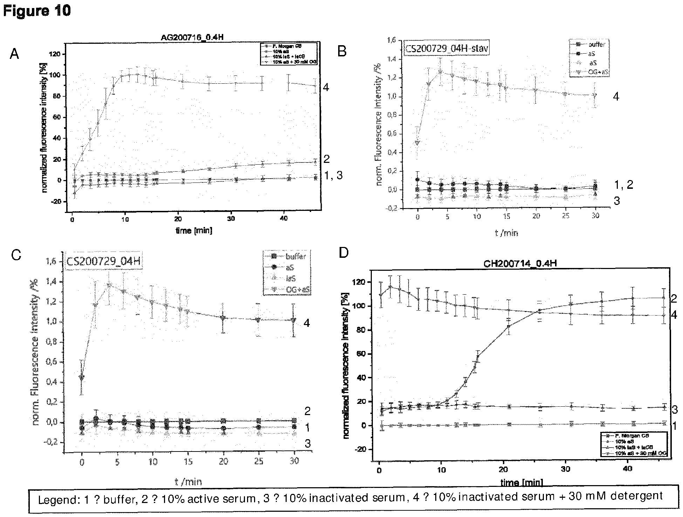

**Figure 10** shows liposome stability in human serum in the absence of complement activators, as described in Example 3. An example of cationic liposomes incubated with 10% human serum is given (Figure 10A). In Figure 10B and C the same liposomes are shown, once with and once without streptavidin coupled to their surface. As seen in the Figure 10D, if the cholesterol amount is chosen too high (e.g. anionic liposomes with 44% of the lipid bilayer being cholesterol), the liposomes are lysed by the complement system.

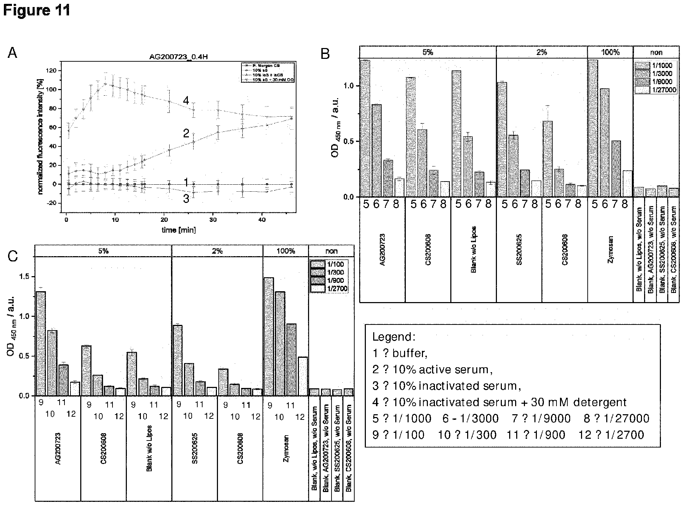

**Figure 11** shows exemplary targeted liposome lysis through LPS modification and complement activation. 'AG' liposomes are modified with 1 mol% LPS, 'SS' liposomes contain 44 mol% cholesterol. Both of these liposomes lead to increased concentrations of C3a and C5a proteins, similar to the positive control Zymosan. In contrast, 'CS' liposomes are stealth and did not trigger the complement system, its signals are similar to the sample containing no liposomes.

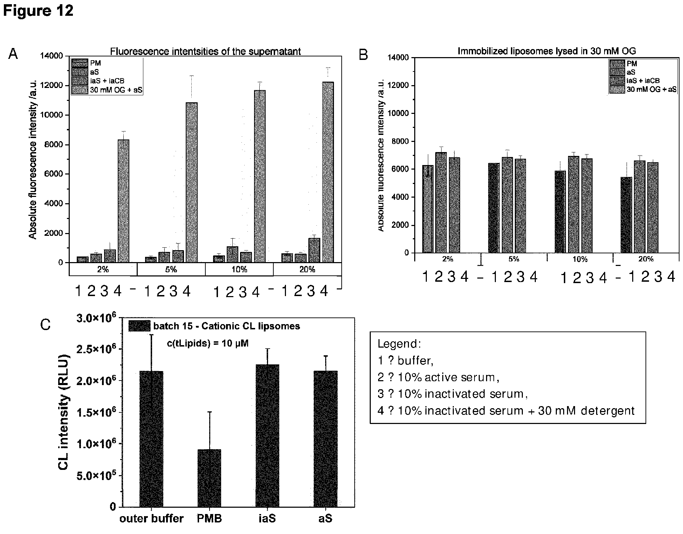

**Figure 12** shows exemplary heterogeneous complement assays, as described in Example 3. A) analysis of the supernatant of SRB containing pegylated cationic liposomes investigated in a heterogeneous complement assay. B) Analysis of remaining, immobilized liposomes. The signals of the supernatant samples are low, whereas the signals obtained from immobilized liposomes are high. In the case of cationic liposomes entrapping 30 mM mCOOH-luminol for chemiluminescence detection, only the signal of the remaining, intact immobilized liposomes is recorded (Figure 12C).

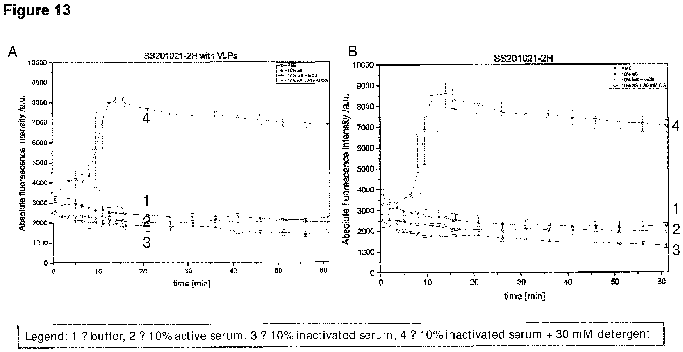

**Figure 13** shows the effect of an exemplary pathogen-mimicking particle, e.g. VLP, on stability of an exemplary pathogen-targeting particle, e.g. liposome, in human serum. The effect of the presence of VLPs on the stability of liposomes in human serum was investigated. VLPs and anionic, pegylated liposomes were allowed to incubate prior to the addition of human serum (Figure 13A). As negative control, the same liposomes were investigated without the addition of VLPs (Figure 13B). As can be seen in Figure 13, liposomes remain stable and are not lysed by the complement system or serum in the presence of VLPs without the presence of a specific interaction.

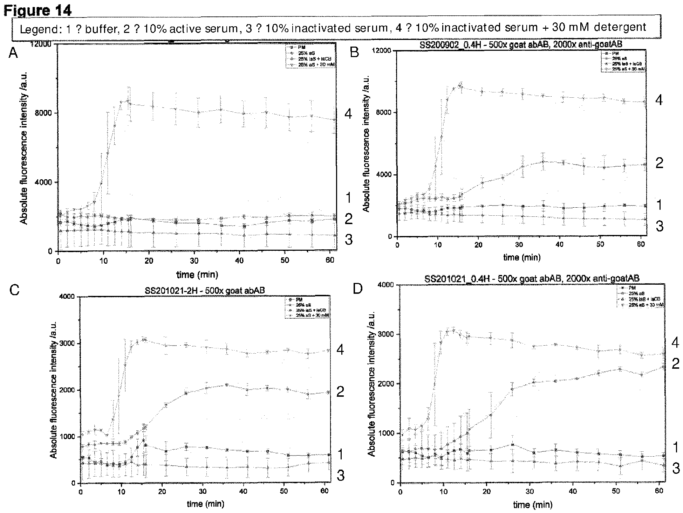

**Figure 14** shows antibody binding to pathogen-targeting particles, e.g. liposomes, and complement activation. Biotinylated anionic liposomes, with (Figure 14C,D) and without pegylation (Figure 14A, B) are lysed, when bound by anti-biotin antibodies derived from goat or donkey. As can be seen, pegylated liposomes are lysed by either antibody, whereas non-pegylated liposomes are only lysed by the donkey-derived antibody.

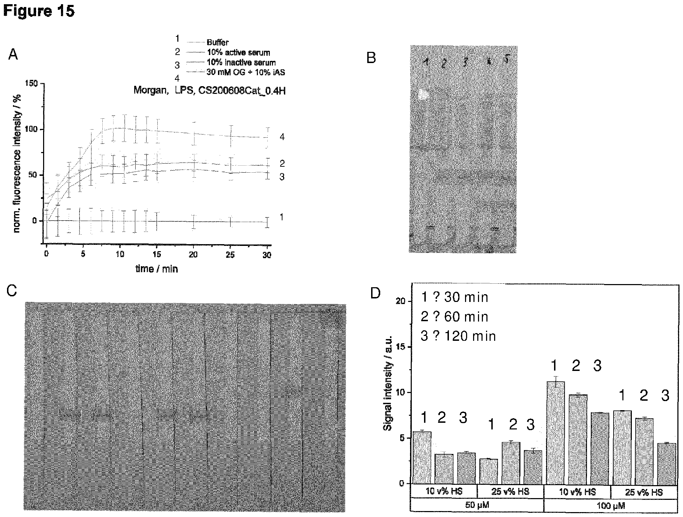

**Figure 15** shows induced liposome lysis independent of the complement system (A) and a detection of liposomes in a LFA (B-D), as described in Example 3.

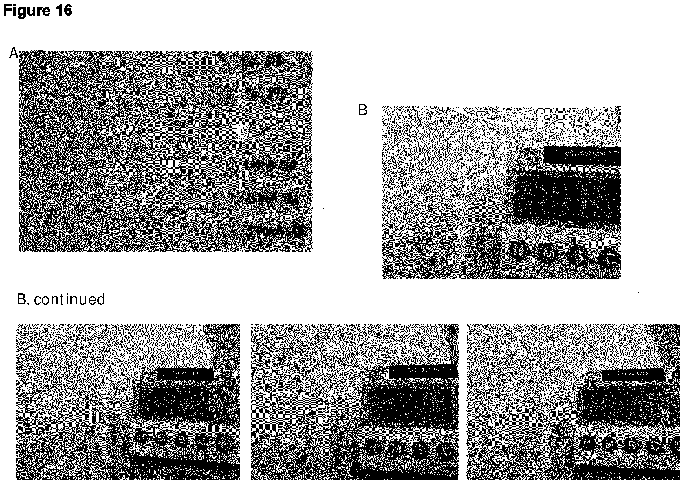

**Figure 16** shows that pH indicator controls are useful in the context of the present invention, e.g. a particle collection can be complemented with a pH indicator, a composition may comprise a pH indicator, the method and/or the use of the invention may comprise using a pH indicator, and a kit and/or a point-of-care device may comprise a pH indicator.

In the following, reference is made to the examples, which are given to illustrate, not to limit the present invention.

### EXAMPLES

### Example 1: Lysis test of different liposome systems in serum

### Materials:

- Homemade activation buffer:
   ∘ 10 mM HEPES, 145 mM NaCl, 67.5 mM CaCl₂, 500 nM, MgCl₂, pH 7.4
- Serum:
   ∘ pooled human complement serum (Innovative Research, Inc.)
   ∘ Serum was inactivated by heating to 65 °C for 30 min in an Eppendorf thermoshaker and by addition of 10 µL 200 mM EDTA containing complement buffer to each well.
   ∘ S serum concentration of 25% was chosen as compromise between fluorescence intensities and high serum consumption.
- Liposomes:
   ∘ anionic biotin containing liposomes extruded through membranes with 1.0 and 0.4 µm pore size **(0.4 µm biotin)** → positive control
   ∘ anionic PEG containing liposomes extruded through membranes with 0.4 and 0.1 µm pore size **(0.1 µm PEG)** → negative control
   ∘ anionic PEG containing liposomes extruded through membranes with 0.4 and 0.1 µm pore size and with 5% inserted LPS **(0.1 µm PEG 5% LPS)**
   ∘ anionic PEG containing liposomes extruded through membranes with 0.4 and 0.1 µm pore size and with 10% inserted LPS **(0.1 µm PEG 10% LPS)**
   ∘ anionic liposomes extruded through membranes with 0.4 and 0.1 µm pore size and with 5% inserted LPS **(0.1 µm non-PEG 5% LPS)**
   ∘ anionic liposomes extruded through membranes with 0.4 and 0.1 µm pore size and with 10% inserted LPS **(0.1 µm non-PEG 10% LPS)**

### Method

As the concentration of liposomes after insertion was not measured by ICP-OES, the concentration was estimated from educts and experience from former insertion reactions. Samples were pipetted to black Nunc Maxisorp microtiterplates by hand. After heating, it was cooled to room temperature and up this gelling and was not hardly pipettible. This lead to very high error bars.
The fluorescence intensity was measured with a BioTek SYNERGY neo2 fluorescence reader. The wavelengths were *λ_{Ex}* = 565 *nm* and λ*_{Em}* = 585 *nm.* The first measurement was conducted after finishing the pipetting process (t=o min). The plate was then incubated twice at 37 °C for 30 min. The second read (t=30 min) took place with the same measurement conditions.

### Results

Figure 7 shows the six tested liposome systems with buffer, 25% active and inactive serum and 10 µM triton X-100. Here, buffer and inactive serum served as negative controls and triton X-100 as positive control. Positive and negative control show expected results for buffer, active serum and triton, whereas the signals for inactive serum are higher than expected and equal the results for active serum. LPS containing liposomes show increased fluorescence upon the buffer negative control and in most cases a similar increase in fluorescence similar to the positive control liposomes (0.4 µm biotin).

### Example 2: Methods

### VLP fabrication

Virus-like particles (VLP) are produced following previously described protocols. In brief, HEK293 cells, once transfected with a lentiviral, RNA- and codon-optimized Gag-gene, readily produce a Gag precursor protein which is targeted to the plasma membrane and released as non-infectious virus-like particle. Such VLPs are - similar to coronaviruses - enveloped by a membrane of cellular origin, and resemble immature lentiviral particles in size (100-150 nm diameter) and shape. If co-expressed in a mammalian cell together with e.g. a viral envelope protein such as the SARS-CoV-2-S-protein or the receptor binding domain, that protein is incorporated into the particle [2].

### Liposome synthesis and modification

Liposomes are synthesized based on previously developed protocols containing 1,2-dipalmitoyl-sn-glycero-3-phosphatidylcholine (DPPC), 1,2-dipalmitoyl-sn-glycero-3-ethylphospho¬choline (EDPPC), 1,2-dipalmitoyl-sn-glycero-3-phospho-(1'-rac-glycerol) (DPPG), 1,2-dipalmitoyl-sn-glycero-3-phosphoethanolamine-N-(biotinyl), cholesterol and pegylated or NTA modified versions of these or similar lipids. A typical protocol is shown here:
DPPC (17.3 mg), EDPPC (4.5 mg) and cholesterol (0.6 mg) were dissolved in chloroform (3 ml) and methanol (0.5 ml) in a 50 ml round bottom flask and sonicated at 60 °C for 1 minute. 2 ml of an aqueous solution containing either sulforhodamine B (SRB, 10 mM, dissolved in 210 mM NaCl, 0.02 M HEPES, pH 7.5) or m-carboxy-luminol (25 mM, dissolved in 0.2 M HEPES, pH 8.5) was added and the mixture sonicated at 60 °C for 4 minutes. The organic solvent was removed by using a rotary evaporator at 60 °C and a pressure of 750 mbar for 40 minutes. Rotary evaporation is a critical step in liposome synthesis where it needs to make sure that the temperature is held above the phase transition temperature of all lipids (here: 60 °C). The solution was vortexed, and another 2 ml of the aqueous solution was added. After vortexing, again the solution was rotated at 60 °C and 750 mbar for 20 minutes and then again at 60 °C and 400 mbar for 20 minutes. This procedure leads to the evaporation of the organic solvent and ensures that most of the aqueous solvent remains in the flask to contain the formed liposomes. The dispersion was being extruded through polycarbonate membranes (1 µm and 0.4 µm) at 60 °C by pushing the syringes back and forth 21 times for each membrane. Excess of the marker molecules was removed by size exclusion chromatography with a Sephadex G-50 column followed by dialysis for 24 h against HEPES-saline-sucrose (HSS) buffer (10 mM HEPES, 200 mM NaCl, 200 mM sucrose, 0.01% NaN₃, pH 7.5, in case of sulforhodamine B) or glycine-NaOH buffer (10 mM glycine, 200 mM NaCl, 114 mM sucrose, 0.01% NaN₃, pH 8.6, in case of m-carboxy-luminol) [3]. Modifications of liposomes are typically obtained by including the appropriate lipid in the lipid mixture. For example lipids modified with biotin, polyethylene glycol, NTA, COOH, NH2. The latter two can then be easily used for additional modifications using standard coupling strategies with EDC/NHS chemistry or through thiocyanides, such as FITC.

### Complement activator generation

Initial studies used different antibodies (generated in mouse, goat, donkey) to bind to biotin and FITC moieties on liposomes. Incubating antibodies with the liposomes prior to the complement assay (see below) was carried out at room temperature or at 37 °C for 30 min - 12 hours.

### Homogeneous complement assay

Liposomes were diluted in complement buffer to a final stock solution of 750 or 100 µM. 10 µL of liposome stock solutions were used to generate 75 or 10 µM in each 100 µL well. Samples were prepared in triplicates. Each assay contained liposomes in complement buffer (CB), 10 %vol. active serum (aS), 10 %vol. heat inactivated serum (iaS) containing 1/10 diluted inactivation buffer and as positive control a detergent containing sample, all prepared in complement buffer. Samples were pipetted to black Corning Costar 96 well microtiter plates by hand. The fluorescence intensity was measured with a BioTek SYNERGY neo2 fluorescence reader. The wavelengths were λ*Ex* = 565 *nm* and *λEm* = 585 *nm.* The gain was set to 125 (for 75 µM samples) or to 150 (for 10 µM samples). The first measurement was conducted after finishing the pipetting process (t=0 min). Plate was then incubated at 37 °C inside the incubator and measured after 30 min incubation. For each measurement, the plate was read 3 consecutive times to relativize the instrument's influence on the signal. If necessary, time resolved measurements were performed starting with a 1.5 minute interval for 15 minutes followed by another 15 minutes with a 5 minute measurement interval. Other measurement settings were kept the same as for endpoint measurements.

### Heterogeneous complement assay

In the case of a heterogeneous complement assay, liposomes were first immobilized onto streptavidin-coated plates (Microcoat Biotechnologie, GmbH). Subsequently, the same assay was performed as described above. Here, both immobilized and lysed liposomes can be investigated.

### Lateral-flow assay

Lateral-flow assays were purchased from Microcoat Biotechnologie, GmbH and contained a streptavidin test line and also a FITC control line. After a complement assays as described above, samples were soaked up by the LFA, followed by a washing buffer.

### Example 3: Results

### Liposome synthesis and characterization

Liposomes are characterized via dynamic light scattering, ICP-OES, fluorescence signal and stability in a complement assay. The table below provides an example of various types of liposomes synthesized (cationic with LPS; cationic, anionic with COOH coupling groups, and pegylated anionic liposomes; in all cases, 2% biotin is presented on the surface), providing information on size, surface charge, and stealthiness (given as % lysis in active serum (aS). All liposomes are stable, with the exception of those modified with LPS, which is a known complement activator and functions consequently as trigger also here. Regarding the zeta potential of the various liposomes, it can be observed the polyethylene glycol shields the surface charge and that also LPS lowers the otherwise expected higher charge (Figure 8).

Other data collected typically include concentration of synthesized liposome solution and SRB concentration. These are plotted as absorbance/concentration values in order to compare the signal amplification enhancement of different types of liposomes. At this point, it can be observed that higher SRB encapsulation efficiency is obtained for cationic liposomes (Figure 8). Further investigations and optimizations are ongoing in order to achieve similar encapsulation efficiencies for all liposomes.

### Protein coupling to liposomes

Streptavidin was coupled to liposomes via standard EDC/NHS chemistry. The successful reaction is demonstrated by immobilizing different concentrations (total lipid in µM) streptavidin-coated liposomes to biotinylated bovine serum albumin (biotin-BSA). Here, controls experiments included the incubation of streptavidin-liposomes to BSA, liposomes to biotinylated BSA and liposomes to BSA. As expected, only the streptavidin-liposomes provided a dose response signal when bound to biotinylated BSA (Figure 9).
Also, biotinylated ACE2 is bound to the above described strepativin-liposomes. Here, liposomes entrapping 30 mM mCOOH-luminol were used. Streptavidin-liposomes and ACE2 were incubated for 1 hour and subsequently allowed to bind to a microtiter plate, into which the receptor binding domain (RBD) of SARS-coronavirus 2 was immobilized. As comparison, the same liposomes, not incubated with ACE2, were also allowed to bind to the RBD. Here, minimal background binding was observed (Figure 9). This indicated clearly that tagging of liposomes with ACE2 was possible and the complex consisting of liposomes-streptavidin-biotin-ACE2 was formed.

### Liposome stability in human serum in the absence of complement activators

Cationic and anionic liposomes, those with pegylation and without, and liposomes with COOH groups, biotin, streptavidin or NTA are not lysed when incubated with human serum for 45- 60 minutes at 37 °C. An example of cationic liposomes incubated with 10% human serum is given (Figure 10A). Negative controls included liposomes incubated in buffer and incubated with inactivated human serum. A positive control were liposomes incubated with detergent and human serum.
Similarly, cationic liposomes (with COOH groups on the outer surface) coupled to streptavidin remain intact when incubated with human serum. In Figure 10B and C shown are the same liposomes, once with and once without streptavidin coupled to their surface. However, if the cholesterol amount is chosen too high (e.g. anionic liposomes with 44% of the lipid bilayer being cholesterol), the liposomes are lysed by the complement system. As seen in the Figure 10D. Here, complement-induced lysis starts after about 11 minutes incubation.

### Targeted liposome lysis through LPS modification and complement activation

When inserting LPS into the lipid bilayer of liposomes, complement-induced lysis can also be observed, as seen in Figure 11A. Cationic liposomes, that otherwise have the same composition as the stealth liposomes shown in Figure 10 are here modified to contain 1 mol% LPS in the lipid bilayer (instead of COOH groups).
The inventors have proven with standard ELISAs that quantify the amount of the proteins C3a (Figure 11B) and C5a (Figure 11C) that indeed their levels are increased in samples that contain liposomes in human serum that specifically activated the complement system. 'AG' liposomes are modified with 1 mol% LPS, 'SS' liposomes contain 44 mol% cholesterol. Both of these liposomes lead to increased concentrations of C3a and C5a proteins, similar to the positive control Zymosan. In contrast, 'CS' liposomes are stealth and did not trigger the complement system, its signals are similar to the sample containing no liposomes.

### Heterogeneous complement assays

In a variation of the homogenous complement assay described above, liposomes are immobilized in a microtiter plate prior to incubation with human serum. Here, liposomes can be immobilized through their biotin (onto streptavidin immobilized within the wells of the microtiter plate), through modification with streptavidin (onto biotinylated BSA immobilized within the wells of the microtiter plate), through their FITC modification (onto anti-FITC antibodies immobilized within the wells of the microtiter plate), etc. This format is advantageous, when e.g. chemiluminescent markers are used, as their detection in serum samples can be difficult and result in unfavorable limits of detection. This format is also good for colorimetric detection. Furthermore, this assay format allows in the case of fluorescence detection the detection of complement/serum-lysed liposomes and the detection of the remaining intact liposomes. Two examples are shown in Figure 12.
In Figure 12, SRB containing pegylated cationic liposomes investigated in a heterogeneous complement assay are shown, with the analysis of the supernatant (Figure 12A) and the analysis of remaining, immobilized liposomes (Figure 12B). In the example shown, liposomes are stealth when incubated with varying concentrations of human serum. That is, in all instances the signals of the supernatant samples are low, whereas the signals obtained from immobilized liposomes are high (and the same for buffer and serum containing samples).
In the case of cationic liposomes entrapping 30 mM mCOOH-luminol for chemiluminescence detection, only the signal of the remaining, intact immobilized liposomes is recorded (Figure 12C). Here, incubation in outer buffer keeps liposomes stable, in Paul Morgan buffer renders a negative control signal. Liposomes incubated in active and inactive human serum (aS, iaS) are stable.

### VLP effect on liposome stability in human serum

The effect of the presence of VLPs on the stability of liposomes in human serum was investigated. VLPs and anionic, pegylated liposomes were allowed to incubate prior to the addition of human serum (Figure 13A). As negative control, the same liposomes were investigated without the addition of VLPs (Figure 13B). As can be seen in Figure 13, liposomes remain stable and are not lysed by the complement system or serum in the presence of VLPs.

### Antibody binding to liposomes and complement activation

Biotinylated anionic liposomes, with (Figure 14C,D) and without pegylation (Figure 14A, B) were shown to be lysed, when bound by anti-biotin antibodies derived from goat or donkey. As can be seen, pegylated liposomes are lysed by either antibody, whereas non-pegylated liposomes are only lysed by the donkey-derived antibody. Without wishing to be bound by any theory, the inventors assume that higher concentrations of the goat-derived antibody will cause the same effect also in the non-pegylated liposomes.

This experiment proves that stealth liposomes can be lysed when another, specifically chosen molecule (here the antibodies) binds to their surface. The antibody therefore resembles the trigger antibody or the trigger-loaded VLP as described in the overall assay format.

### Induced liposome lysis independent of complement system

So far, complement activation for the lysis of liposomes was favored. However, it was also found that the presence of some trigger molecules in solution may cause liposome lysis independent of the complement system, as it also takes place in inactivated serum. Shown in Figure 15A are examples of liposomes modified with 5% LPS. It is therefore also possible to envision trigger molecules, that are independent from the normal complement system pathways and still result in the successful lysis of liposomes, when they come in contact with them (as envisioned in this invention).

### Detection of liposomes in LFA

Biotinylated cationic and anionic liposomes can be captured in a testline in which streptavidin is immobilized (Figure 15B). Shown is the binding of cationic liposomes in dependence of the concentration of CaCl₂ in the buffer solutions. Increased salt concentrations promote better binding of biotinylated liposomes and streptavidin. Similarly, when the liposomes are modified with FITC or digoygenin, they can be capture through an anti-FITC antibody or through anti-dig antibodies (Figure 15C, strips 1-6 with anti-FITC, strips 7-9 with anti-dig, strip 10 control with liposomes without FITC or digoxygenin). Quantification of liposomes specifically lysed through complement activity (here, high cholesterol content anionic liposomes are used) is also possible. Here it is shown that longer incubation times of liposomes with human serum leads to a lower signal in the test line, indicating that more liposomes were lysed (Figure 15D). Of specific interest here are the samples containing 100 µM liposomes incubated with 10 or 25% human serum. It is also seen that the higher human serum content leads to more liposome lysis. When lower concentrations of liposomes are used (50 µM), already lower incubation times lead to a loss in signal.

### Possibilities for pH indicator controls in LFA

PH indicators can be added to the sample. Their appearance on a waste pad impregnated with a different pH will render the waste pad colorful indicating the complete run of an assay (Figure 16A). Alternatively, dyes can be added, with the draw back that they may increase the background signal in the testline and control line themselves (Figure 16A). Different substances were added to the anionic running buffer: 1 - 1 µL bromothymol blue, 2 - 5 µL bromothymol blue, 3 - no additive, 4 - 100 µM SRB, 5 - 250 µM SRB, 6 - 500 µM SRB. Alternatively, a pH indicator can be added as an additional control line (Figure 16B). As soon as it is in contact with the sample solution, it will change color and migrate onto the waste pad. Thus, disappearance of the color in the control line or appearance of the color on the waste pad can function as measurement point.

### REFERENCES

[1] Peaper et al. Handbook of Clinical Neurology, vol. 123, Neurovirology, chapter 5.
[2] Demi L, Schirmbeck R, Reimann J, Wolf H, Wagner R. Recombinant human immunodeficiency Pr55gag virus-like particles presenting chimeric envelope glycoproteins induce cytotoxic T-cells and neutralizing antibodies. Virology. 18. August 1997;235(1):26-39.
[3] Hofmann, C., Kaiser, B., Märkl, S., Duerkop, A., Baeumner, A.J., Cationic liposomes for generic signal amplification strategies in bioassays' Analytical and Bioanalytical Chemistry, 412(14), 3383-3393. Front cover, DOI: 10.1007/S00216-020-02612-W.
[4] Ludwig C and Wagner R, Virus-like particles-universal molecular toolboxes, Current Opinion in Biotechnology 2007, 18:537-545.

The features of the present invention disclosed in the specification, the claims, and/or in the accompanying figures may, both separately and in any combination thereof, be material for realizing the invention in various forms thereof.

## Claims

1. A particle collection for detecting a pathogen-neutralizing molecule, preferably antibody, comprising
- a pathogen-mimicking particle, preferably a non-infectious modified pathogen, a pathogen-like particle such as a virus-like particle (VLP), a fusion protein, and/or a nanoparticle selected from silica nanoparticles, polymeric nanoparticles, e.g. PLA nanoparticles, organic nanoparticles, and inorganic nanoparticles, wherein said pathogen-mimicking particle comprises at least one biomarker, preferably surface biomarker, of said pathogen;
- a pathogen-targeting particle, preferably a liposome, wherein said pathogen-targeting particle comprises
at least one pathogen-targeting molecule, wherein said pathogen-targeting molecule is capable of binding, preferably specifically binding, to said biomarker of said pathogen; and
a marker;
- optionally, a complement activating agent,
wherein, preferably, said complement activating agent recognizes and/or binds to said pathogen-mimicking particle, and/or
said complement activating agent is incorporated in said pathogen-mimicking particle.

2. The particle collection according to claim 1, wherein said at least one biomarker of said pathogen-mimicking particle is presented on a surface, preferably outer surface, of said pathogen-mimicking particle and/or is integrated in said pathogen-mimicking particle,
wherein, optionally, said biomarker is associated with said pathogen-mimicking particle via a transmembrane domain of said biomarker, a linker, a GPI anchor, a PEG, an enzymatic linkage such as a sortase linkage, a homo- or heterobifunctional crosslinker, or natural or non-natural aminoacid(s).

3. The particle collection according to claim 1 or 2, wherein said biomarker is a SARS-CoV2 biomarker, preferably any of a S (spike) protein, E (envelope) protein, M (membrane) protein, N (nucleocapsid) protein, an HIV envelope biomarker, influenza hemagglutinin, influenza neuraminidase, influenza M protein, an Ebola biomarker, a Marburg virus glycoprotein, a Lassa virus glycoprotein, a Herpes virus biomarker, a bacterial surface biomarker, or a fragment thereof, more preferably a SARS-CoV-2-S protein or a fragment thereof such as the receptor-binding domain thereof; and/or wherein said pathogen-mimicking particle is a SARS-CoV-2-like particle.

4. The particle collection according to any one of the foregoing claims, wherein said pathogen-targeting particle comprises a liposome; and/or
wherein said pathogen-targeting particle, preferably liposome, is biotinylated, PEGylated, streptavidinylated, anionic, cationic, zwitterionic, coated with other stabilizing molecules such as proteins or polymers, or functionalized with a coupling group such as NH2, COOH, OH, NTA, short peptides, polysaccharides, or short nucleic acid strands; and/or
wherein said pathogen-targeting particle has an average size in the range of from 1 nm to 600 nm, preferably 50 nm to 300 nm, more preferably 75 nm to 250 nm, e.g. 150 nm.

5. The particle collection according to any one of the foregoing claims, wherein said pathogen-targeting molecule is any of a receptor, ligand, enzyme, and a fragment thereof, preferably transmembrane protease serine 2 (TMPRSS2), ACE2, CD4, CCR5, CXCR4, or a fragment thereof, more preferably ACE2 or a fragment thereof.

6. The particle collection according to any one of the foregoing claims, wherein said pathogen-targeting particle comprises a marker selected from a fluorescent marker, preferably sulforhodamine B or carboxyfluorescein, an electrochemical marker, preferably potassium hexaferricyanide or hexaferrocyanide, or ruthenium hexamine, a colorimetric marker, preferably sulforhodamine B, a chemiluminescent marker, preferably m-COOH luminol, an electrochemiluminescent marker, preferably mCOOH-luminol or ruthenium bipyridyl, a bioluminescent marker such as GFP, or an enzyme substrate such as glucose, lactate or ATP, an enzyme such as glucose oxidase, alkaline phosphatase, or peroxidase, or DNA molecules,
wherein, optionally, said marker is contained inside the pathogen-targeting particle, preferably in an inner space of said pathogen-targeting particle.

7. The particle collection according to any one of the foregoing claims,
wherein said complement activating agent comprises any of an antibody, a peptide, e.g. binding peptide, a protein, e.g. Protein A or G, streptavidin, biotin, a lectin, a carbohydrate, cholesterol, PEG, a nucleic acid, an aptamer, a complement component, e.g. a C3b-fragment, a microbial component, e.g. LPS, a component of an apoptotic cell, a pentraxin, and fragments, combinations, or multimers thereof;
wherein, optionally, said complement activating agent further comprises a complement activating moiety,
wherein, preferably, said complement activating moiety is selected from LPS, a Fc domain or a Fc-multimer, a Fc CH3 domain or a CH3-multimer, a carbohydrate, and a complement component, e.g. a C3b-fragment.

8. The particle collection according to any one of the foregoing claims, wherein said complement activating agent recognizes and/or binds to said at least one biomarker of said pathogen-mimicking particle; and/or
wherein said complement activating agent recognizes and/or binds to a target, e.g. a peptide, on said pathogen-mimicking particle other than the at least one biomarker; and/or
wherein said complement activating agent is incorporated in said pathogen-mimicking particle such that said complement activating agent and/or a complement activating moiety comprised by said complement activating agent is/are presented on a surface of said pathogen-mimicking particle.

9. A composition comprising a particle collection of any one of claims 1 to 8, wherein said composition comprises any of a pathogen-mimicking particle, a pathogen-targeting particle, and a complement activating agent, preferably comprises a pathogen-mimicking particle and a pathogen-targeting particle, and/or a pathogen-mimicking particle, a pathogen-targeting particle, and a complement activating agent.

10. A method of detecting a pathogen-neutralizing molecule, preferably antibody, using a particle collection of any one of claims 1 to 8 or a composition of claim 9, preferably an *in vitro* method of detecting a pathogen-neutralizing antibody of a patient to a pathogen or a method for screening pathogen-targeting compound(s), comprising the steps:
i) contacting a sample with said pathogen-mimicking particle,
wherein, optionally, said sample is a sample obtained from a patient, preferably a serum sample, or said sample is a target compound to be tested or compound library to be screened,
ii) optionally, incubating said sample with said pathogen-mimicking particle, e.g. for 0 to 120 minutes at a temperature of from 4 to 40°C,
iii) adding said pathogen-targeting particle comprising a marker thereto, preferably a liposome,
iv) optionally, incubation, e.g. for 0 to 120 minutes at a temperature of from 15 to 40 °C, preferably 20 to 37 °C, such as 22 °C,
v) optionally, adding said complement activating agent and/or standardized blood components thereto,
vi) optionally, incubation, e.g. for 10 to 120 minutes at a temperature of from 20 to 40 °C, preferably 37 °C,
vii) detecting a signal of said marker;
wherein, optionally, said method comprises a step of immobilizing said pathogen-targeting particle.

11. The method according to claim 10, wherein said detecting is performed using any of colorimetric, fluorescent, chemiluminescent, electrochemiluminescent, electrochemical, bioluminescent, and/or visual detection, optionally detection using a cell phone; and/or
wherein said marker is selected from a fluorescent marker, preferably sulforhodamine B or carboxyfluorescein, an electrochemical marker, preferably potassium hexaferricyanide or hexaferrocyanide, or ruthenium hexamine, a colorimetric marker, preferably sulforhodamine B, a chemiluminescent marker, preferably m-COOH luminol, an electrochemiluminescent marker, preferably mCOOH-luminol or ruthenium bipyridyl, a bioluminescent marker such as GFP, or an enzyme substrate such as glucose, lactate or ATP, an enzyme such as glucose oxidase, alkaline phosphatase, or peroxidase, or DNA molecules.

12. The method according to claim 10 or 11, further comprising determining a presence of a pathogen-neutralizing molecule, preferably antibody, if a signal detected in step iv) is increased compared to a reference signal and/or reference value.

13. A kit for detecting a pathogen-neutralizing molecule, preferably antibody, comprising a particle collection of any one of claims 1 to 8 or a composition of claim 9, further comprising any of
- an auxiliary agent, preferably any of a buffer, a solvent, standardized blood components, e.g. standardized human blood components, a standardized serum, inactivated serum, an indicator dye, and a detergent;
- optionally, a test strip;
- optionally, an electrode;
- optionally, a microtiterplate;
- optionally, instructions for determining a presence of a pathogen-neutralizing molecule, preferably antibody, if a signal of a marker is increased compared to a reference signal and/or reference value.

14. A point-of-care device, preferably an electrochemical and/or lateral-flow assay point-of-care device, comprising
- an inlet for receiving at least one sample, preferably a serum sample;
- a contacting unit for contacting said sample(s) with any of a pathogen-mimicking particle, a pathogen-targeting particle comprising a marker, a complement activating agent, and combinations thereof, preferably for contacting said sample(s) with a particle collection of any one of claims 1 to 8 or a composition of claim 9;
- optionally, a test line with an immobilized and/or adsorbed recognition element, for example streptavidin, polystreptavidin, antibodies against digoxygenin or FITC, biotinylated BSA, or an affinity recognition element such as avidin, Protein A or G, a polymer, a lectin, a protein or polymer labeled with haptens, charged molecules, NTA, or DNA molecules;
- optionally, a control line containing an immobilized and/or adsorbed control recognition element, for example streptavidin, polystreptavidin, antibodies against digoxygenin or FITC, biotinylated BSA, or an affinity recognition element such as avidin, Protein A or G, a polymer, a lectin, a protein or polymer labeled with haptens, charged molecules, NTA, or DNA molecules;
- optionally, a pH indicator, e.g. a pH indicator dye, for example impregnated in a sample pad or conjugate pad or waste pad;
- optionally, a waste pad impregnated with a buffer for providing a color change of a pH indicator once the pH indicator reaches the waste pad;
- a detecting unit for detecting a signal of said marker, preferably a detecting unit for colorimetric, fluorescent, chemiluminescent, electrochemiluminescent, electrochemical, bioluminescent, and/or visual detection, optionally detection using a cell phone;
- optionally, an output unit for outputting a result obtained from said detecting.

15. Use of a particle collection of any one of claims 1 to 8 or a composition of claim 9 in a method of detecting a pathogen-neutralizing molecule, preferably antibody, preferably a method of any one of claims 10 to 12.
